# NEUE EUROPÄISCHE PATENTSCHRIFT

(11) **EP 2 772 304 B2**
(45) Veröffentlichungstag und Bekanntmachung des Hinweises auf die Entscheidung über den Einspruch: **07.08.2024**
(45) Hinweis auf die Patenterteilung: 10.05.2017
(21) Anmeldenummer: 13005208.7
(22) Anmeldetag: 18.05.2012
(51) Int. Cl.: B01J 14/00, C07C 201/16, C07C 205/06

(54) **Vorrichtungen zur Aufreinigung von Nitrierprodukten**
Devices for the purification of nitration products
Dispositifs de nettoyage de produits de nitruration

(30) Priorität: 19.05.2011 DE 102011102059
(43) Veröffentlichungstag der Anmeldung: 03.09.2014
(62) Teilanmeldung aus: 12723831.9
(73) Patentinhaber: Josef Meissner GmbH & Co. KG, 50968 Köln (DE)
(72) Erfinder: Pöhlmann, Jürgen, D - 50969 Köln (DE); Hermann, Heinrich, D - 50858 Köln (DE); Händel, Mirko, D - 53819 Neunkirchen-Seelscheid (DE); Gebauer, Jürgen, D - 53840 Troisdorf (DE)
(74) Vertreter: Von Rohr Patentanwälte Partnerschaft mbB

(56) Entgegenhaltungen:
- EP-A1- 0 771 783
- EP-A2- 0 279 312
- DE-A1- 2 151 206
- DE-B- 1 222 904
- US-A- 3 221 064
- US-A- 4 482 769
- US-A- 4 597 875

## Beschreibung

Die vorliegende Erfindung betrifft das technische Gebiet der Nitrierung, insbesondere der Herstellung nitrierter aromatischer organischer Verbindungen (synonym nachfolgend auch als "Nitroaromaten", "Nitrierprodukte" oder dergleichen bezeichnet) und deren Aufreinigung nach ihrer Herstellung.

Insbesondere betrifft die vorliegende Erfindung eine Vorrichtung bzw. Anlage zur Entfernung von Verunreinigungen (wie z. B. insbesondere nichtumgesetzten Edukten, Reaktionsnebenprodukten, Nitriersäure und deren Umsetzungsprodukten, wie Stickoxiden oder Salpetriger Säure etc.) aus bei der Nitrierung von nitrierbaren aromatischen Verbindungen nach Abtrennung der Nitrierendsäure anfallenden nitrierten Rohprodukten durch Behandlung mit einem Waschmedium. Mit anderen Worten betrifft die vorliegende Erfindung somit eine Vorrichtung bzw. Anlage zur Aufreinigung von bei der Nitrierung von nitrierbaren aromatischen Verbindungen nach Abtrennung der Nitrierendsäure anfallenden nitrierten Rohprodukten.

Die erfindungsgemäße Vorrichtung bzw. Anlage ist insbesondere zur Durchführung eines entsprechenden Verfahrens zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium geeignet.

Aromatische Nitroverbindungen, wie z. B. Nitrobenzol (MNB), Mononitrotoluol (MNT), Dinitrotoluol (DNT), Trinitrotoluol (TNT), Nitrochlorbenzol (MNCB) etc., welche durch Umsetzung eines entsprechenden Aromaten, wie z. B. Benzol, Toluol, Xylol, Chlorbenzol, Dichlorbenzolen etc., mit Salpetersäure - direkt oder in Gegenwart von Schwefelsäure als Katalysator und wasserbindendes Mittel - hergestellt werden, müssen vor ihrer Weiterverarbeitung einer mehrstufigen Wäsche und zusätzlichen Reinigungsstufen unterworfen werden, um die in den rohen Nitroaromaten noch gelösten oder suspendierten Verunreinigungen, wie z. B. Schwefelsäure, Salpetersäure, Nitrose, Nitrophenole, Nitrokresolen etc., die beispielsweise als Mono-, Dinitro- und Trinitroverbindungen vorliegen können, und sonstige Oxidationsprodukte, wie z. B. Nitrobenzoesäuren und Abbauprodukte aus der Zersetzung der Nitrophenole, bzw. die nichtumgesetzten Aromaten oder nicht gewünschte Isomere, wie z. B. bei der TNT-Herstellung, im rohen Gemisch der Nitroaromaten zu entfernen.

Üblicherweise besteht die Wäsche der rohen Nitroaromaten zur Entfernung der darin gelösten und suspendierten Säuren des Nitriergemisches, der Nitrophenole und anderer saurer und sonst noch mit dem Waschmittel extrahierbaren Verunreinigungen aus drei Schritten (siehe z. B. F. Meissner et al., Industrial and Engineering Chemistry, Vol. 46, Seiten 718 bis 724 (1954); Ullmanns Enzyklopädie der Technischen Chemie, 4. Auflage, Bd. 17, Seiten 384 bis 386; H. Hermann et al., "Industrial Nitration of Toluene to Dinitrotoluene", ACS Symposium Series 623 (1996), Seiten 234 bis 249, Editors: L. F. Albright, R. V. C. Carr, R. J. Schmitt; US 6 288 289 B1; EP 1 816 117 B1). Als Waschmedium wird dafür üblicherweise Wasser eingesetzt, wobei die Wäsche üblicherweise als flüssig/flüssig-Wäsche (d. h. bei Temperaturen, bei denen der zu waschende Nitroaromat als Flüssigkeit vorliegt) durchgeführt wird.

Diese dreistufige Wäsche umfasst üblicherweise die folgenden Schritte:
1. Eine saure Wäsche mit Wasser zur Entfernung der gelösten und suspendierten Mineralsäuren, wie z. B. Schwefelsäure, Salpetersäure und Nitrose ("saure Wäsche").
2. Eine basische bzw. alkalische Wäsche in Gegenwart einer Base ("Alkaliwäsche"), wie z. B. Natriumcarbonat (Soda), Natriumbicarbonat, Natriumsulfit, Natriumhydrogensulfit, Ammoniak, Natronlauge, Kalilauge etc. (siehe z. B. US 4 482 769 A, US 4 597 875 A oder US 6 288 289 B1), zur Entfernung der im rohen Nitroaromaten gelösten schwach aciden Verunreinigungen, wie der Nitrophenole, Nitrokresole, Nitrobenzoesäuren, Abbauprodukte aus der oxidativen Zersetzung der Phenole oder von aliphatischen oder cyclischen Kohlenwasserstoffen etc., wie z. B. Oxalsäure etc., oder den asymmetrischen Isomeren beim TNT ("basische Wäsche").
3. Eine Neutralwäsche zur Entfernung der Restspuren von Alkali und der weiteren Reduzierung der noch in Spuren im Produkt verbliebenen Verunreinigungen ("neutrale Wäsche").

Ziel dieser Waschschritte ist es, neben einem reinen Produkt möglichst wenig Abwasser pro Tonne Produkt zu erhalten, in welchem die ausgewaschenen Verunreinigungen so vorliegen, dass ihre Entsorgung kostengünstig durchgeführt werden kann.

Zur Minimierung der für diese Wäsche benötigten Wassermengen kann die Wäsche beispielsweise im Gegenstrom derart erfolgen, dass das zur Neutralwäsche benutzte Wasser nach Zusatz von Basen in der Alkaliwäsche eingesetzt wird (vgl. z. B. A. B. Quakenbush et. al., The Olin Dinitrotoluene (DNT) Process, Polyurethanes World Congress 1993, Publish.: Technomic Lancaster, Seiten 484 bis 488) oder aber dass bei der sauren Wäsche mit einer minimalen Menge an Wasser derart gewaschen wird, dass eine konzentrierte Säure erhalten wird, welche direkt oder nach weiterer Aufkonzentrierung in die Nitrierung zurückgeführt werden kann.

So werden in der EP 0 279 312 B1, der EP 0 736 514 B1 und der EP 1 780 195 B1 Verfahren beschrieben, mit denen die in den Nitroaromaten nach der Nitrierung noch suspendierten und gelösten Mineralsäuren, wie Schwefelsäure, Salpetersäure und Nitrose, mehrstufig und selektiv ausgewaschen und in die Nitrierung zurückgeführt werden, so dass kein Abwasser mehr aus der sauren Wäsche anfällt und entsorgt werden muss.

Es sind aber auch Verfahren bekannt geworden, bei denen - um die zu behandelnde Abwassermenge zu minimieren - keine saure Wäsche durchgeführt wird, sondern nur eine alkalische und Neutralwäsche, wie beispielsweise in Kirk-Othmer, Encyclopedia of Chemical Technology, 4. Ed., Vol. 17, Seiten 136 bis 138, oder in der US 4 091 042 A beschrieben.

Neben der Minimierung der Abfallströme ist es weiterhin das Ziel, den für die Wäsche benötigten technischen Aufwand zu minimieren (z. B. dadurch, dass die für die Wäschen eingesetzte Technologie nicht nur an die Waschstufe, sondern auch an das zu waschende Produkt spezifisch angepasst wird).

Als Waschapparate werden für die Wäsche der zu reinigenden Nitroaromaten auf den einzelnen Waschstufen üblicherweise so genannte Mixer-Settler (Mischer-Scheider) (vgl. z. B. EP 1 593 654 A1) eingesetzt, bei denen der Mischteil üblicherweise ein Rührkessel ist (vgl. z. B. Ullmann's Encyclopedia of Industrial Chemistry, 5. Ed., Vol. B 3, Seiten 6.19 bis 6.21; M. Baerns et al., Technische Chemie, Verlag Wiley-VCH 2006, Seiten 352/352). So wird bereits in der deutschen Patentschrift DE 1 135 425 eine Anordnung von Mischer und Scheider beschrieben, die es erlaubt, auch bei Raumtemperatur kristalline Nitroaromaten, wie z. B. DNT, TNT oder NCB, bei erhöhten Temperaturen unter Minimierung des Beheizungsaufwandes flüssig zu waschen. Aber auch Kreiselpumpen und statische Mischer sind als Mischer eingesetzt worden (vgl. z. B. die Druckschriften US 3 221 064 A oder EP 1 816 117 B1).

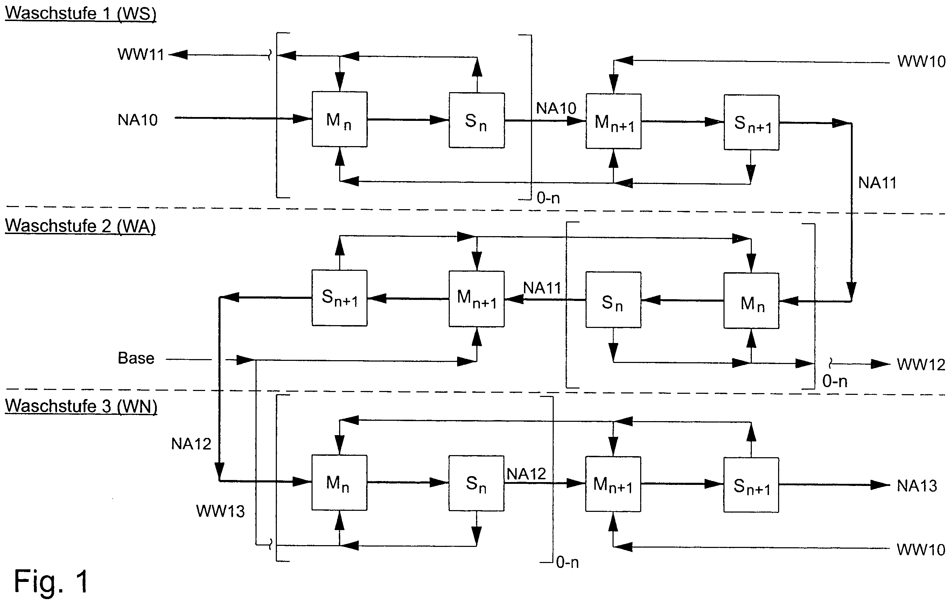

Der Einsatz der Mixer/Settler-Technologie (Mischer/Scheider-Technologie) (vgl. z. B. Fig. 1) ist aber aufwendig und teuer. Durch den nicht vermeidbaren Schlupf bei kontinuierlich betriebenen Rührkesseln als Mixer bzw. Mischer ist es vor allem bei der Entfernung der Nitrophenole oder Nitrokresole, wenn diese in hohen Konzentrationen im rohen Nitroaromaten vorliegen, notwendig, mehrstufig und möglichst im Gegenstrom zu arbeiten, um den für die Weiterverarbeitung des Nitroaromaten gewünschten niedrigen Gehalt an Verunreinigungen zu erhalten (z. B. einen Gehalt an Nitrophenolen von weniger als 10 ppm, bevorzugt 2 bis 3 ppm). Auch eine Wäsche in mehrstufigen Extraktionskolonnen ist technisch aufwendig und teuer und nicht sehr effektiv. Außerdem ist die Erzeugung großer Austauschflächen für ein zweiphasiges Gemisch in kurzer Zeit für einen effektiven Massentransfer ("*mass transfer*"), gefolgt von einer schnellen chemischen Umsetzung, weder in einem Rührkessel noch in Extraktionskolonnen realisierbar.

In J. M. Coulson, F. E. Warner, "A Problem in Chemical Engineering Design: The Manufacture of Mononitrotoluene", einer Veröffentlichung von "The Institution of Chemical Engineers", 56, Victoria Street, London, S.W. I, 1949, Seiten 25/26, wird eine dreifache Wäsche des MNT mit einem Wäscher von Typ Holley-Mott (Mixer/Settler) beschrieben, wobei die saure und die alkalische Wäsche in jeweils mindestens zwei Stufen im Gegenstrom durchgeführt wird, um eine ausreichende Entfernung der im MNT gelösten bzw. suspendierten Säuren und Nitrokresole zu erreichen.

In der kanadischen Patentschrift CA 1 034 603 wird eine vierstufige saure Wäsche im Gegenstrom vorgeschlagen, um die im rohen DNT gelöste und suspendierte Salpeter- und Schwefelsäure auszuwaschen.

In der US 4 091 042 A wird zur Entfernung aller saurer Komponenten aus rohem Nitrobenzol, wie mitgerissener Schwefelsäure und der im Nitroaromaten gelösten Dinitrophenole und Pikrinsäure von bis zu 2.000 ppm, eine vierstufige Wäsche mit Soda im Gegenstrom beschrieben, um die gewünschte Reinheit zu erhalten.

In der EP 1 816 117 A1 wird eine vierstufige Neutralwäsche im Gegenstrom unter Einsatz von vier Rührwerksbehältern und den zugehörigen Trennapparaten beschrieben (sogenannte "Mixer/Settler-Technologie"), um den noch zu hohen Gehalt an Nitrophenolen nach der alkalischen Wäsche von ca. 50 ppm auf einen Restgehalt von ca. 2 ppm zu reduzieren. Aber auch bei Ersatz der Rührwerksbehälter durch Kreiselpumpen als Mischorgan werden immer noch drei Stufen benötigt, um einen Restgehalt an Nitrophenolen im resultierenden Nitrobenzol von 3 ppm zu erhalten.

Die US 4 994 242 A offenbart, dass statische Mischer im technischen Maßstab allein als Mischorgan in zweiphasigen Systemen nicht geeignet sind, um eine optimale Dispergierung der zwei nicht miteinander mischbaren Phasen ineinander zu erzeugen. So wird in der EP 1 816 117 B1 der Einsatz eines statischen Mischers für die alkalische Wäsche beschrieben; das damit behandelte Nitrobenzol enthält noch mehr als 50 ppm an Nitrophenolen, die durch eine aufwendige mehrstufige Neutralwäsche auf ca. 2 ppm reduziert werden müssen.

Wie bereits in der EP 1 780 195 B1 für eine saure Wäsche erläutert, ist die Wäsche von Nitroaromaten ein komplexer Vorgang. Neben der Erzeugung einer genügend großen Austauschfläche zwischen Organphase und Waschphase (üblicherweise Wasser), um einen optimalen Übergang des aus der Organphase zu entfernenden Verunreinigung zu erreichen, hängt die Effektivität einer Waschstufe von den Verteilungsgleichgewichten der Verunreinigung zwischen Organphase und Waschmedium und auch davon ab, ob die aus der Organphase extrahierte Verunreinigung als solche in dem Waschmedium stabil ist oder durch eine nachfolgende Umsetzung dem Verteilungsgleichgewicht entzogen wird.

So reagiert Nitrose nach dem Übergang aus der Organphase in die wässrige Phase mit Wasser unter Disproportionierung zu Salpetersäure und NO nach Gleichung (1):

(1) 3 NO₂ (= 3/2 N₂O₄) + H₂O → 2 HNO₃ + NO

Sowohl der Übergang der Nitrose aus der Organphase, wahrscheinlich als Dimeres, als auch die Umsetzung der Nitrose (als N₂O₄) mit dem Wasser sind vergleichsweise langsam ablaufende Reaktionen im Vergleich zu einer Neutralisation, so dass für die Entfernung der Nitrose aus der Organphase durch eine Wäsche mit nachfolgender chemischer Umsetzung Zeit benötigt wird.

Bei Säuren hingegen, wie Schwefelsäure, Salpetersäure oder den schwach sauren Nitrophenolen, ist die im Waschwasser eintretende Dissoziation der Säuren in Hydroniumionen und die zugehörigen Anionen (Gleichung 2) oder die in Gegenwart von Alkali erfolgende Neutralisation (Gleichung 3) ein sehr schneller Vorgang, durch welchen die ausgewaschenen Verunreinigungen dem Verteilungsgleichgewicht zwischen Nitroaromaten und Waschwasser entzogen werden und sich in anionischer Form nur noch im Waschwasser befinden.

(2) H₂SO₄ + H₂O → H₃O⁺ + HSO₄⁻

(3) NO₂Ar-OH + NaOH → NO₂Ar-O⁻ Na⁺+ H₂O

Durch diese schnelle Neutralisation von Anionen bildenden Stoffen im alkalischen Waschmedium ist zu erwarten, dass die Extraktion dieser Stoffe aus der Organphase im Wesentlichen massentransferkontrolliert verläuft und die Wäsche im Wesentlichen den gleichen kinetischen Gesetzmäßigkeiten folgt wie eine Mononitrierung, z. B. wie die Nitrierung von Benzol zu Nitrobenzol.

Die aus dem Stand der Technik bekannten Verfahren und Anlagen zur Aufreinigung nitrierter Rohprodukte arbeiten oftmals nicht mit hoher Effizienz oder aber nicht in zufriedenstellender Weise. Bisweilen sind hiermit auch übermäßig komplexe Verfahrensabläufe oder Arbeitsgänge verbunden, und es werden oftmals nicht die gewünschten Reinheiten erreicht, zumindest nicht mit vertretbarem Aufwand.

Dokument DE 21 51 206 A1 betrifft ein Verfahren und eine Vorrichtung zum Vermischen von Flüssigkeiten, wobei die Vermischung von Flüssigkeiten unter Verwendung von Strahldüsen durchgeführt wird, welche in einen Mischreaktor hineinragen.

Dokument DE 12 22 904 B betrifft ein Verfahren zur Reinigung von nitrophenolhaltigen aromatischen Nitroverbindungen, wobei die zu reinigenden aromatischen Nitroverbindungen einer Wäsche mit Wasser unterzogen und nachfolgend entweder unmittelbar oder nach Vermischung mit dem gleichen Volumen einer 0,1- bis 2-gewichtsprozentigen wässrigen Natronlauge versetzt und anschließend über ein alkalisch gestelltes Anionenaustauscherharz geleitet werden.

Dokument US 3 221 064 A betrifft ein Verfahren zur Reinigung von Dinitrotoluolen, wobei die Dinitrotoluole in einer Kreiselpumpe mit Wasser sowie wässriger Natriumhydroxidlösung emulgiert werden und nach jedem Waschschritt die Abtrennung des entsprechenden Waschmediums erfolgt.

Dokument EP 0 279 312 A betrifft ein Verfahren zur Abtrennung von Schwefelsäure und Salpetersäure aus bei der Nitrierung von Toluol erhaltenen Dinitrotoluolgemischen, wobei die Dinitrotoluole mit Wasser vermischt und die sich danach abscheidende, Schwefel- und Salpetersäure enthaltende wässrige Phase abgetrennt wird.

Dokument US 4 597 875 A betrifft ein Verfahren zur Entfernung von aus bei der Herstellung von Nitroaromaten anfallenden Nitrokresol- und Pikrinsäureverunreinigungen aus Abwässern, wobei die Verunreinigungen über mehrere Waschschritte kumuliert, mit Säure behandelt und anschließend die ausgefällten organischen Rückstände verbrannt werden.

Dokument US 4 492 860 A betrifft ein Verfahren zur Aufreinigung von Dinitrotoluol, wobei das Dinitrotoluol mit einer wässrigen basischen Phase gewaschen wird, um die Dinitroorthokresol-Verbindungen zu entfernen, das Dinitroorthokresol jedoch in der organischen Phase zu belassen.

Der vorliegenden Erfindung liegt daher die Aufgabe zugrunde, eine Vorrichtung bzw. Anlage zur Entfernung von Verunreinigungen aus bei der Nitrierung von nitrierbaren aromatischen Verbindungen nach Abtrennung der Nitrierendsäure anfallenden nitrierten Rohprodukten bereitzustellen, wobei die zuvor geschilderten, im Zusammenhang mit dem Stand der Technik auftretenden Probleme und Nachteile zumindest weitgehend vermieden oder aber wenigstens abgeschwächt werden sollen.

Insbesondere ist eine Aufgabe der vorliegenden Erfindung darin zu sehen, eine zur Durchführung eines entsprechenden Verfahrens geeignete Vorrichtung bzw. Anlage bereitzustellen, mit welcher eine effiziente Aufreinigung der nitrierten Rohprodukte, wie sie aus der Nitrierung von nitrierbaren aromatischen Verbindungen nach Abtrennung der so genannten Nitrierendsäuren hervorgehen, ermöglicht werden sollen.

Weiterhin besteht eine Aufgabe der vorliegenden Erfindung darin, die Wäsche der nach Abtrennung der Nitrierendsäure resultierenden rohen Nitroaromaten, in denen signifikante Mengen an Verunreinigungen, wie beispielsweise noch mitgerissene Nitriersäure, gelöste Schwefelsäure, Salpetersäure, Nitrose, Nitrophenole, Nitrobenzoesäuren, Abbauprodukte aus dem oxidativen Abbau von Nitrophenolen etc., vorliegen können, quasi einstufig in jedem Waschschritt derart durchzuführen, dass im gewaschenen Nitroaromaten der Nitrophenolgehalt möglichst gering ist (z. B. bei Nitrobenzol aus einer adiabatischen Nitrierung mit ursprünglich ca. 2.000 ppm Di- und Trinitrophenolen der Gehalt an Nitrophenolen nach der alkalischen Wäsche unter 50 ppm, bevorzugt unter 10 ppm, und nach der Neutralwäsche unter 2 ppm liegt) und dass der Aufwand und die Kosten dafür deutlich geringer sind als bei den bisher genutzten Verfahren und Vorrichtungen des Standes der Technik.

Die zuvor geschilderte Aufgabenstellung wird erfindungsgemäß durch eine Vorrichtung bzw. Anlage nach Anspruch 1 gelöst; weitere, vorteilhafte Weiterbildungen und Ausgestaltungen dieses Erfindungsaspekts sind Gegenstand der diesbezüglichen Unteransprüche.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf die übrigen Erfindungsaspekte entsprechend gelten.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Dies vorausgeschickt, wird im Folgenden die vorliegende Erfindung näher beschrieben.

Mit der erfindungsgemäßen Vorrichtung bzw. Anlage lässt sich somit ein Verfahren zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium durchführen, wobei das Verfahren die folgenden Verfahrensschritte umfasst:
- In einem ersten Verfahrensschritt (a) werden zunächst die nitrierten Rohprodukte mit einem Waschmedium in Kontakt gebracht und die nitrierten Rohprodukte und das Waschmedium ineinander verteilt derart, dass eine Dispersion, insbesondere Emulsion, resultiert (d. h. mit anderen Worten wird in diesem ersten Verfahrensschritt (a) eine Dispersion bzw. Emulsion von nitrierten Rohprodukten einerseits und Waschmedium andererseits hergestellt), und
- in einem zweiten Verfahrensschritt (b) wird nachfolgend die resultierende Dispersion, insbesondere Emulsion, in einen Rohrreaktor einspeist, so dass während des Durchtritts der Emulsion durch den Rohrreaktor die in den nitrierten Rohprodukten anfänglich vorhandenen Verunreinigungen entfernt werden und/oder so dass während des Durchtritts der Emulsion durch den Rohrreaktor die in den nitrierten Rohprodukten anfänglich vorhandenen Verunreinigungen in das Waschmedium überführt bzw. hierdurch neutralisiert werden.

Das vorstehende Verfahren eignet sich somit in hervorragender Weise zur Aufreinigung von bei der Nitrierung von nitrierbaren aromatischen Verbindungen nach Abtrennung der Nitrierendsäure anfallenden nitrierten Rohprodukten.

Das Prinzip des vorstehenden Verfahrens besteht also darin, die aus der Nitrierung stammenden, noch signifikante Mengen an Verunreinigungen enthaltenden rohen Nitroaromaten - nach Abtrennung der Nitrierendsäure (z. B. in einem Scheider) - zunächst mit einem Waschmedium in Kontakt zu bringen und die Mischung aus aufzureinigenden Nitroaromaten und Waschmedium in eine Emulsion bzw. Dispersion zu überführen und nachfolgend die resultierende Emulsion bzw. Dispersion in einen Rohrreaktor einzuspeisen, damit die in den aufzureinigenden Nitroaromaten anfänglich vorhandenen Verunreinigungen in das Waschmedium überführt bzw. hierdurch neutralisiert werden und auf diese Weise ein aufgereinigter Nitroaromat entsteht.

Denn, wie die Anmelderin in vollkommen überraschender Weise herausgefunden hat, führt die Verwendung eines Rohrreaktors - in Kombination mit einer vorgeschalteten Dispergier- bzw. Emulgiereinrichtung - dazu, dass eine besonders gute Durchmischung und besonders innige und feine Verteilung von Waschmedium einerseits und aufzureinigenden Nitroaromaten andererseits erreicht werden kann, so dass auf diese Weise die Verunreinigungen sozusagen in einem einzigen Verfahrensschritt (nämlich im Rahmen der Rohrreaktorbehandlung) vollständig bzw. zumindest im Wesentlichen vollständig entfernt werden können.

Im Unterschied zum Stand der Technik werden also weitergehende, komplexe Verfahrensschritte zur Aufreinigung des rohen Nitroaromaten in effizienter Weise vermieden, ohne dass Qualitätseinbußen bei der Aufreinigung des rohen Nitroaromaten hinzunehmen wären.

Überraschenderweise gewährleistet der erfindungsgemäß für die Behandlung des rohen Nitroaromaten mit dem Waschmedium eingesetzte Rohrreaktor eine derart innige und feine Verteilung von rohem Nitroaromaten einerseits und Waschmedium andererseits, dass im Rahmen der Rohrreaktorbehandlung gemäß Verfahrensschritt (b) sämtliche bzw. zumindest im Wesentlichen sämtliche Verunreinigungen in das Waschmedium überführt bzw. hierdurch neutralisiert werden, so dass sie anschließend (d. h. nach Abschluss des Verfahrensschritts (b)) zusammen mit dem Waschmedium von dem dann aufgereinigtem Nitroaromaten abgetrennt werden können.

Es hat sich nämlich in überraschender Weise gezeigt, dass es im Rahmen der vorliegenden Erfindung möglich ist, eine Wäsche von Nitroaromaten erfolgreich quasi einstufig - auch bei hoher Belastung mit Verunreinigungen, wie Nitriersäure, Nitrophenolen und Nitrokresolen - durchzuführen, und zwar durch eine einfache und kostengünstige Kombination von Strahlmischern, aber auch anderen Dispergierorganen, wie z. B. Kreiselpumpen, mit zusätzlichen Einrichtungen, wie statischen Mischern, Blenden etc. in Rohrreaktoren allein oder noch in Verbindung mit Rührkesseln, die es gestatten, eine genau definierte Mischenergie in das Gemisch der nicht miteinander mischbaren Phasen einzutragen. Die dadurch herstellbaren Emulsionen aus der zu reinigenden Organphase im Waschmedium (O/W-Typ) oder des Waschmediums in der Organphase (W/O-Typ) liefern die für eine effektiven und optimalen Massentransfer benötigten Grenzfläche zwischen zu waschendem Nitroaromat und Waschmedium.

Was die Herstellung der Emulsion bzw. Dispersion in Verfahrensschritt (a) anbelangt, so erfolgt diese im Allgemeinen mittels einer geeigneten Dispergier- bzw. Emulgiereinrichtung, insbesondere mittels eines geeigneten Mischorgans.

Im Rahmen der vorliegenden Erfindung kann als Dispergier- bzw. Emulgiereinrichtung (d. h. insbesondere als vorzugsweise erste Dispergier- bzw. Emulgiereinrichtung), insbesondere als Mischorgan, beispielsweise ein Rührkessel, ein Strahlmischer (Jet-Mixer bzw. *Jet-Mixing-Device*) oder eine Pumpe, insbesondere eine Kreiselpumpe, eingesetzt werden.

Gemäß einer Ausführungsform wird als Dispergier- bzw. Emulgiereinrichtung, insbesondere als Mischorgan, eine Pumpe, insbesondere eine Kreiselpumpe, im Rahmen von Verfahrensschritt (a) eingesetzt.

Gemäß einer alternativen, bevorzugten Ausführungsform wird im Rahmen von Verfahrensschritt (a) als Dispergier- bzw. Emulgiereinrichtung, insbesondere als Mischorgan, ein so genannter Strahlmischer (synonym auch als "Jet-Mixer" oder als "*Jet-Mixing-Device*" bezeichnet) eingesetzt. Bei dem erfindungsgemäß eingesetzten Strahlmischer handelt es sich insbesondere um eine Einrichtung, welche einen (zentralen) Treibstahl in einem den (zentralen) Treibstrahl umgebenden Medium (z. B. Ringstrahl) erzeugt.

Als Strahlmischer können alle Arten von Strahlmischern eingesetzt werden, die es gestatten, mittels des zentralen Treibstrahls als Freistrahl, der sowohl aus dem Waschmedium wie auch dem zu waschenden Nitroaromaten bestehen kann, mit hoher Relativgeschwindigkeit den zu waschenden Nitroaromaten oder das Waschmedium derart einzudüsen, dass entweder der zu waschende Nitroaromat im Waschmedium oder das Waschmedium im zu waschenden Nitroaromaten als Emulsion mit großer Grenzfläche verteilt wird. Einrichtungen dieser Art sind beispielsweise in Ullmann's Encyclopedia of Industrial Chemistry, 2003, 5. Ed., Vol. B 4, Seiten 87/88 und 565 bis 571, oder aber in Perry's Chemical Engineers' Handbook, McGraw-Hill Book Company, 1984, 6. Auflage, Seiten 5-21 bis 5-23 oder aber in der deutschen Offenlegungsschrift DE 2 151 206 beschrieben.

Dabei kann der (zentrale) Treibstrahl im Strahlmischer das Waschmedium und das umgebende Medium der aufzureinigende nitrierte Roharomat sein; alternativ kann aber auch der (zentrale) Treibstrahl durch das aufzureinigende nitrierte Rohprodukt und das den (zentralen) Treibstrahl umgebende Medium durch das Waschmedium gebildet sein. Beide alternativen Ausführungsformen führen zu dem gewünschten Ergebnis.

Besonders gute Ergebnisse im Hinblick auf die Aufreinigung des aufzureinigenden Roharomaten werden (unabhängig davon, ob der zentrale Treibstrahl durch das Waschmedium oder aber durch das aufzureinigende nitrierte Rohprodukt gebildet wird) erhalten, wenn das Verhältnis der Geschwindigkeiten zwischen dem zentralen Treibstrahl einerseits und dem den zentralen Treibstrahl umgebendem Medium (z. B. Ringstrahl) im Strahlmischer im Bereich von 1 : 5 bis 30 : 1, bevorzugt im Bereich von 1 : 2 bis 20 : 1, besonders bevorzugt im Bereich von 1 : 1 bis 10 : 1, eingestellt wird. Auf diese Weise wird eine besonders innige und feine Verteilung von Waschmedium einerseits und Rohprodukt andererseits und folglich eine besonders effiziente Aufreinigung erzielt.

Die Fließgeschwindigkeit der Waschemulsion nach dem Strahlmischer im nachfolgenden Rohrreaktor liegt insbesondere im Bereich von 0,1 bis 15,0 m/s, bevorzugt im Bereich von 0,5 bis 10 m/s.

Gemäß einer Ausführungsform der vorliegenden Erfindung kann es vorgesehen sein, dass die im Verfahrensschritt (a) eingesetzte Dispergiereinrichtung, insbesondere das Mischorgan, dem Rohrreaktor vorgeschaltet, insbesondere unmittelbar vorgeschaltet, ist. Gemäß einer besonderen Ausgestaltung dieser Ausführungsform kann es vorgesehen sein, dass die Dispergier- bzw. Emulgiereinrichtung, insbesondere das Mischorgan, in den Rohrreaktor übergeht.

Gleichermaßen ist es jedoch auch möglich, dass die Dispergiereinrichtung, insbesondere das Mischorgan, in den Rohrreaktor integriert ist bzw. Bestandteil des Rohrreaktors ist. Zu diesem Zweck kann beispielsweise die Dispergiereinrichtung in dem oberen bzw. stromaufwärts befindlichen Teil des Rohrreaktors angeordnet sein. Eine solche Ausführungsform ist insbesondere dann möglich, wenn die Dispergiereinrichtung, insbesondere das Mischorgan, als so genannter Strahlmischer ausgebildet ist.

Erfindungsgemäß ist es vorgesehen, dass der Rohrreaktor zur Durchführung von Verfahrensschritt (b) mit Mischelementen zum Eintrag von zusätzlicher Mischenergie ausgestattet ist, auf diese Weise können besonders gute Reinigungsergebnisse erzielt werden, da durch die zusätzlichen Mischelemente eine noch weiter verbesserte, besonders innige Verteilung von Waschmedium einerseits und aufzureinigendem Roharomaten andererseits erreicht wird. Bei den Mischelementen kann es sich insbesondere um Bleche, insbesondere Prall- oder Umlenkbleche, Blenden, statische Mischer, Stromteiler oder dergleichen handeln. Erfindungsgemäß ist es bevorzugt, wenn 1 bis 15, insbesondere 2 bis 15, bevorzugt 2 bis 10, besonders bevorzugt 2 bis 5, Mischelemente in dem Rohrreaktor vorliegen.

Erfindungsgemäß ist es vorgesehen, dass die durch die im Rohrreaktor vorgesehenen Mischelemente eine Mischenergie (d.h. eine volumenbezogene Mischenergie) insgesamt von 10 bis 1.000 Joule/Liter, bevorzugt 10 bis 500 Joule/Liter, besonders bevorzugt 20 bis 200 Joule/Liter, eingetragen wird. Mit anderen Worten wird eine Mischenergie (d.h. eine volumenbezogene Mischenergie) von 10 bis 1.000 Joule/Liter, bevorzugt 10 bis 500 Joule/Liter, besonders bevorzugt 20 bis 200 Joule/Liter, insgesamt eingetragen.

Besonders gute Ergebnisse werden erfindungsgemäß auch dadurch erzielt, dass die Mischelemente derart ausgebildet sind, dass der Druckabfall pro Mischelement 0,1 bar bis 3,0 bar, bevorzugt 0,3 bis 1,5 bar, besonders bevorzugt 0,3 bis 0,8 bar, beträgt.

Was die Verweilzeit der Emulsion von Waschmedium einerseits und Roharomaten andererseits im Rohrreaktor im Rahmen von Verfahrensschritt (b) anbelangt, so kann diese in weiten Bereichen variieren. Besonders bevorzugt ist es, wenn die Verweilzeit im Rohrreaktor 0,1 bis 120 Sekunden, bevorzugt 0,1 bis 60 Sekunden, besonders bevorzugt 1 bis 30 Sekunden, beträgt. Auf diese Weise werden besonders gute Waschergebnisse erzielt, da zum einen eine ausreichende Mindestverweilzeit, andererseits jedoch auch ein ökonomischer Durchsatz gewährleistet wird.

Im Rahmen der Aufreinigung sind auch das Masse- und Phasenverhältnis zwischen aufzureinigenden nitrierten Rohprodukten einerseits und Waschmedium andererseits von Bedeutung, welche jeweils in weiten Bereichen variieren können.

Besonders gute Ergebnisse werden erhalten, wenn das Masseverhältnis zwischen aufzureinigenden nitrierten Rohprodukten einerseits und Waschmedium (d. h. frisch zudosiertem Waschmedium) andererseits im Bereich von 200 : 1 bis 1 : 10, bevorzugt im Bereich von 100 : 1 bis 1 : 5, besonders bevorzugt im Bereich von 10 : 1 bis 1 : 2, eingestellt wird.

Gleichermaßen werden besonders gute Ergebnisse dann erhalten, wenn das Phasenverhältnis (d. h. insbesondere das Phasenverhältnis im Waschapparat) zwischen aufzureinigenden nitrierten Rohprodukten einerseits und Waschmedium andererseits im Bereich von 25 : 1 bis 1 : 5, insbesondere im Bereich von 10 : 1 bis 1 : 2, bevorzugt im Bereich von 5 : 1 bis 1 : 1, eingestellt wird. Die Einstellung des Phasenverhältnisses kann insbesondere durch eine Kreisführung des Waschmediums nach Phasentrennung erzielt werden. Dies gewährleistet einerseits eine optimale Austauschfläche zwischen Organphase und Waschmedium und andererseits eine möglichst kurze Zeit für die Phasentrennung im Phasentrennapparat.

Die Wäsche der Nitroaromaten wird üblicherweise als flüssig/flüssig-Wäsche (d. h. bei Temperaturen, bei denen der zu waschende bzw. aufzureinigende Nitroaromat - ebenso wie das Waschmedium - als Flüssigkeit vorliegt) durchgeführt.

Was das eingesetzte Waschmedium anbelangt, so ist dieses unter Verfahrensbedingungen, insbesondere bei Temperaturen ab 5 °C, insbesondere bei Temperaturen ab 25 °C, und Atmosphärendruck, flüssig ausgebildet. Bevorzugt wird ein wässrig basiertes Waschmedium, vorzugsweise Wasser, eingesetzt.

Je nach Phasenverhältnis im Waschapparat wird dabei der zu waschende Nitroaromat im Waschmedium als Öl-in-Wasser-Emulsion (O/W-Emulsion) oder das Waschmedium im zu waschenden Aromat als Wasser-in-Öl-Emulsion (W/O-Emulsion) dispergiert.

Die Effizienz des Waschmediums kann noch dadurch gesteigert werden, dass dem Waschmedium mindestens eine Base zugesetzt werden kann. Die Base kann insbesondere ausgewählt sein aus der Gruppe von anorganischen Hydroxiden, Carbonaten, Hydrogencarbonaten, Sulfiten, Hydrogensulfiten und Ammoniak sowie deren Mischungen oder Kombinationen, bevorzugt aus der Gruppe von Natronlauge, Kalilauge, Natriumcarbonat, Kaliumcarbonat, Natriumhydrogencarbonat, Kaliumhydrogencarbonat, Ammoniak, Ammoniumcarbonat, Natriumsulfit und Natriumhydrogensulfit sowie deren Mischungen oder Kombinationen.

Die bei einer alkalischen Wäsche eingesetzte Menge an Alkali sollte insbesondere derart hoch sein, dass nicht nur alle Säuren quantitativ zu ihren Salzen umgesetzt werden können, sondern es sollte insbesondere ein Überschuss an Base eingesetzt werden, damit der pH-Wert in der Waschlauge derart hoch ist, dass auch schwache Säuren, wie Mononitrophenole quantitativ ausgewaschen werden können.

Der Gehalt an Alkali kann insbesondere 0,01 mol/l bis 0,4 mol/l, bevorzugt 0,02 mol/l bis 0,2 mol/l, mindestens aber das Doppelte der für die Neutralisation aller Nitrophenole benötigten Menge, betragen.

Besonders gute Ergebnisse werden dann erhalten, wenn der Gehalt an Base im Waschmedium 0,01 bis 0,4 mol/l, bevorzugt 0,02 bis 0,2 mol/l, beträgt.

Insbesondere sollte der Gehalt an Base im Waschmedium mindestens das Doppelte der für die Neutralisation aller als Verunreinigungen enthaltener Nitrophenole theoretisch benötigten Alkalimenge betragen.

Wie zuvor ausgeführt, sollte also das Phasenverhältnis von zu waschendem Nitroaromat und frisch zudosiertem Waschmedium vorteilhafterweise 200 : 1 bis 1 : 10, bevorzugt 100 : 1 bis 1 : 5, besonders bevorzugt 10 : 1 bis 1 : 2, betragen. Durch eine Kreisführung des Waschmediums nach Phasentrennung kann dabei im Waschapparat ein Phasenverhältnis zwischen zu waschendem Nitroaromat und Waschmedium von 25 : 1 bis 1 : 5, insbesondere 10 : 1 bis 1 : 2, besonders bevorzugt 5 : 1 bis 1 : 1, eingestellt werden, um einerseits eine optimale Austauschfläche zwischen Organphase und Waschmedium zu erzeugen und andererseits die Zeit für die Phasentrennung im Phasentrennapparat so kurz wie möglich zu halten.

Je nach Phasenverhältnis im Waschapparat wird dabei der zu waschende Nitroaromat im Waschmedium als Öl-in-Wasser-Emulsion (O/W- Emulsion) oder das Waschmedium im zu waschenden Aromat als Wasser-in-Öl-Emulsion (W/O-Emulsion) dispergiert (vgl. obige Ausführungen).

Als Treibstrahl dient dabei, in Abhängigkeit vom gewählten Phasenverhältnis, entweder der zu waschende Aromat oder das Waschmedium, um den gewünschten Emulsionstyp einzustellen.

Die Fließgeschwindigkeit der Waschemulsion nach dem Strahlmischer im nachfolgenden Rohrreaktor kann insbesondere im Bereich von 0,1 bis 15,0 m/s, bevorzugt 0,5 bis 10 m/s, liegen.

Das Verhältnis der Geschwindigkeit zwischen zentralem Strahl und umgebenden Medium bewegt dabei - wie zuvor angeführt - sich zwischen 1 : 5 bis 30 : 1, bevorzugt 1 : 2 bis 20 : 1 und besonders bevorzugt zwischen 1 : 1 bis 10 : 1.

Um die Koaleszenz der Waschemulsion nach kurzer Zeit und damit eine nicht vollständige Extraktion der aus dem zu reinigenden Nitroaromaten zu entfernenden Verunreinigung zu verhindern, ist es vorteilhaft, durch zusätzlichen Eintrag von Mischenergie die Waschemulsion solange stabil zu halten, bis alle Verunreinigungen aus dem Nitroaromaten ausgewaschen sind und durch weitere Umsetzungen im Waschmedium an der Rückextraktion in den zu waschenden Nitroaromaten gehindert werden. Diese zusätzliche Mischenergie wird in das Gemisch der zwei nicht miteinander mischbaren Phasen durch eine Einspeisung in einen Reaktor mit zusätzlichen Mischeinrichtungen, bevorzugt in einen Rohrreaktor ohne Rückvermischung, eingebracht, wobei im Rohrreaktor durch zusätzliche Mischelemente, über den Rohrreaktor verteilt, wie Blenden, Umlenkbleche, Strömungsbrecher, Statikmischer oder andere statischen Mischelemente, die Emulsion vom Typ O/W bzw. W/O aufrechterhalten wird. Bevorzugterweise können 1 bis 15, insbesondere 2 bis 15, bevorzugt 2 bis 10 und besonders bevorzugt 2 bis 5 Mischelemente in dem Rohrreaktor vorliegen, wobei der Strahlmischer als Mischelement mitzählt.

Die insgesamt einzutragende volumenbezogene Mischenergie beträgt 10 bis 1000 J/l, bevorzugt 10 bis 500 J/1 und besonders bevorzugt 20 bis 200 J/1.

Der Druckverlust pro Mischelement, beträgt 0,1 bis 3,0 bar, bevorzugt 0,2 bis 1,5 bar und besonders bevorzugt 0,2 bis 0,8 bar, um die Anzahl der im Rohrreaktor benötigten zusätzlichen Mischelemente so niedrig wie möglich und die Verweilzeit in der Phasentrenneinrichtung so kurz wie möglich zu halten.

Die Verweilzeit in dem Rohrreaktor zur Abtrennung von Säuren, gefolgt von einer schnellen Weiterreaktion, wie z. B. einer Neutralisation, aus dem zu waschenden Nitroaromaten, wie Salpetersäure, Schwefelsäure, Mono-, Di- und Trinitrophenolen und Kresolen, Nitrobenzoesäuren etc., bei einer Wäsche mit Alkali, wie z. B. Natronlauge, Soda, Bicarbonat, Ammoniak, Kalilauge etc., sollte nicht mehr als 0,1 bis 120 Sekunden, bevorzugt 0,1 bis 60 Sekunden, besonders bevorzugt 1 bis 30 Sekunden, betragen.

Zur Entfernung von Verunreinigungen aus dem zu waschenden Nitroaromaten mit hohen Verteilungskoeffizienten zugunsten des zu waschenden Nitroaromaten, hohen Massentransferwiderständen in der Organphase und langsamen Weiterreaktionen der extrahierten Verunreinigung im Waschmedium, wie z.B. Nitrose oder Stickstoffdioxid, sollte die Verweilzeit im nachfolgenden Reaktor an diese Verhältnisse angepasst werden (wie z. B. durch eine Kombination der vorstehend beschriebenen Einrichtungen zur Erzeugung einer optimalen Waschemulsion mit Rührkesseln, um die notwendige Verweilzeit zu erzeugen). Gemäß einer besonderen Ausführungsform des vorstehenden Verfahrens wird dies insbesondere erreicht durch eine Kombination der vorstehend beschriebenen Einrichtungen zur Erzeugung einer optimalen Waschemulsion mit Rührkesseln, um die notwendige Verweilzeit für den Phasentransfer und die nachfolgende Umsetzung sicherzustellen.

Wie zuvor bereits ausgeführt, sollte die bei einer alkalischen Wäsche eingesetzte Menge an Alkali derart hoch sein, dass nicht nur alle Säuren quantitativ zu ihren Salzen umgesetzt werden können, sondern es sollte ein Überschuss an Base eingesetzt werden, damit der pH-Wert in der Waschlauge derart hoch ist, dass auch schwache Säuren, wie Mononitrophenole quantitativ ausgewaschen werden können. Wie zuvor angeführt, sollte dabei der Gehalt an Alkali insbesondere 0,01 mol/l bis 0,4 mol/l, bevorzugt 0,02 mol/l bis 0,2 mol/l, mindestens aber das Doppelte der für die Neutralisation aller Nitrophenole benötigten Menge, betragen.

Die am Ende der Mischstrecke vorliegende Emulsion kann beispielsweise in einem Phasentrennapparat (z. B. Scheider bzw. Settler) wieder in die einzelnen Phasen getrennt werden. Das Waschmedium mit den darin enthaltenen Verunreinigungen kann entweder als Abwasser einer Abwasserbehandlung zugeführt oder im Gegenstrom in die vorgeschaltete Waschstufe eingebracht werden.

Der gewaschene Nitroaromat kann entweder in die nachfolgende Waschstufe eingespeist oder am Ende der Wäsche direkt zur Weiterverarbeitung oder in ein Zwischenlager transferiert werden.

Als Phasentrennapparat können alle Arten von statischen Scheidern zum Einsatz kommen, aber auch dynamische Scheider, wie Zentrifugalseparatoren. Die Scheidezeit der Emulsion Nitroaromat/Waschmedium hängt außer vom Emulsionstyp (W/O oder O/W) und der eingetragenen Mischenergie zusätzlich auch von dem Überschuss an Base im Waschmedium, der nicht zur Neutralisation benötigt wird, ab. Bei Eintrag gleicher Mischenergie sinkt die Scheidezeit deutlich mit zunehmender Basekonzentration im Waschmedium. Zur Beschleunigung der Phasentrennung können aber auch oberflächenaktive Mittel oder auch mechanische Trennhilfen, wie Packungen, Trennbleche etc., eingesetzt werden. Auch durch einen auf den Nitroaromaten und Emulsionstyp abgestimmten Abstand zwischen den einzelnen Mischelementen kann die Phasentrennung beschleunigt werden.

Was die aufzureinigenden nitrierten Rohprodukte anbelangt, so sind diese im Allgemeinen unter Verfahrensbedingungen, insbesondere bei Temperaturen ab 5 °C, insbesondere bei Temperaturen ab 25 °C, und Atmosphärendruck, flüssig ausgebildet. Insbesondere stammen die aufzureinigenden nitrierten Rohprodukte aus der Nitrierung von ein- oder mehrkernigen Aromaten, insbesondere aus der Nitrierung von Benzol, Toluol, Xylol oder halogenierten Aromaten, wie insbesondere chlorierten Benzolen.

Bei den aufzureinigenden nitrierten Rohprodukten handelt es sich insbesondere um gegebenenfalls halogenierte Mono-, Di- und Trinitroaromaten, wie z. B. Nitrobenzol (MNB), Mononitrotoluol (MNT), Dinitrotoluol (DNT), Trinitrotoluol (TNT), Nitrochlorbenzol (MNCB) oder dergleichen.

Im Allgemeinen schließt sich dem Verfahrensschritt (b) eine Abtrennung der von den Verunreinigungen befreiten nitrierten Produkte vom Waschmedium an. Diese Abtrennung erfolgt im Allgemeinen mittels einer geeigneten Scheideeinrichtung (Scheider bzw. Settler).

Weiterhin kann es gemäß einer besonderen Ausführungsform des vorstehenden Verfahrens vorgesehen sein, dass das aus dem Rohrreaktor austretende Gemisch aus gereinigten nitrierten Produkten und Waschmedium, insbesondere noch vor Abtrennung der von den Verunreinigungen befreiten nitrierten Produkte vom Waschmedium, zunächst in einen Rührkessel überführt wird. Auf diese Weise wird die Kontakt- und/oder Verweilzeit zwischen aufzureinigenden Nitrierprodukten einerseits und Waschmedium andererseits in effizienter Weise verlängert, so dass gegebenenfalls noch nicht ausgewaschene Verunreinigungen in das Waschmedium überführt bzw. hierdurch neutralisiert werden.

Gemäß einer vorteilhaften Ausführungsform des vorstehenden Verfahrens ist es vorgesehen, dass das Waschmedium, insbesondere nach Abtrennung der von den Verunreinigungen befreiten nitrierten Produkte vom Waschmedium, rezykliert wird. Auf diese Weise wird eine effiziente Wäsche bzw. Kreisführung ermöglicht und die Menge an Waschmedium auf ein Minimum reduziert.

Gegebenenfalls können nach der Wäsche bzw. nach Abtrennung des Waschmediums (z. B. nach einer Trennung der Waschemulsion in einem statischen Scheider oder durch einen Zentrifugalseparator) gegebenenfalls noch vorhandene Restmengen bzw. Spuren an Wasser, insbesondere suspendiertes und/oder gelöstes Wasser, durch Trocknung aus dem aufgereinigten Nitroaromaten entfernt werden.

Das vorstehende Verfahren eignet sich zur Durchführung der sauren Wäsche und/oder der basischen Wäsche und/oder der neutralen Wäsche der nitrierten Rohprodukte. Das vorstehende Verfahren kann also in allen drei vorgenannten Waschschritten zum Einsatz kommen. Gleichermaßen ist es aber auch möglich, das vorstehende Verfahren nur für eine oder zwei Waschstufen zu verwenden, beispielsweise nur für die saure Wäsche oder aber nur für die basische Wäsche oder aber nur für die neutrale Wäsche. In dieser Hinsicht ist das vorstehende Verfahren flexibel einsetzbar.

Wie zuvor geschildert, ist das vorstehende Verfahren mit einer Vielzahl von Vorteilen und Besonderheiten verbunden, von denen nachfolgend einige Vorteile und Besonderheiten - jedoch nicht abschließend und in nicht beschränkender Weise - aufgeführt werden sollen:

Insbesondere ermöglicht das vorstehende Verfahren eine effiziente Aufreinigung von aus bei der Nitrierung von nitrierbaren aromatischen Verbindungen nach Abtrennung der Nitrierendsäure anfallenden nitrierten Rohprodukten mit nur geringer Komplexität und großer Verfahrensökonomie wie Verfahrenseffizienz.

Der erfindungsgemäß eingesetzte Rohrreaktor ermöglicht eine effiziente und innige Verteilung von Waschmedium einerseits und nitrierten Roharomaten andererseits ineinander, so dass keine weiteren Waschschritte oder anderweitige Behandlungsschritte erforderlich sind. Die Wasch- bzw. Behandlungseffizienz kann noch dadurch gesteigert werden, dass in dem Rohrreaktor zusätzliche Mischelemente vorgesehen sind, wie zuvor geschildert, welche die Durchmischung noch weiterführend verbessern.

Der erfindungsgemäß für die Aufreinigung zum Einsatz kommende Rohrreaktor kann gleichermaßen bei der vorangehenden Nitrierung als Reaktionsbehältnis eingesetzt werden, so dass keine zusätzliche Apparatur für die Aufreinigung der nitrierten Rohprodukte eingesetzt zu werden braucht.

Der erfindungsgemäß für die Aufreinigung der rohen Nitrierprodukte zum Einsatz kommende Rohrreaktor ermöglicht die Erzeugung großer Austauschflächen für ein zweiphasiges Gemisch aus Waschmedium einerseits und nitrierten Roharomaten andererseits, so dass auf diese Weise ein effektiver Massentransfer und eine schnelle Überführung der Verunreinigungen in das Waschmedium bzw. im Fall saurer Verbindungen eine schnelle Neutralisation gewährleistet wird.

Weiterhin ermöglicht die vorstehende Verfahrensführung eine schnelle und gleichsam effiziente Beseitigung der aus der Nitrierung stammenden Verunreinigungen aus den nitrierten Rohprodukten, wobei das Waschmedium nach der Behandlung der nitrierten Roharomaten ohne Weiteres rezykliert bzw. im Kreis gefahren werden kann.

Gegenstand der vorliegenden Erfindung ist eine Vorrichtung (Anlage) zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium, wobei sich die erfindungsgemäße Vorrichtung insbesondere zur Durchführung eines wie zuvor geschilderten Verfahrens eignet,
wobei die Vorrichtung die folgenden Einrichtungen aufweist:
(a) mindestens eine Dispergiereinrichtung, insbesondere mindestens ein Mischorgan, zum Inkontaktbringen und Emulgieren von aufzureinigenden nitrierten Rohprodukten einerseits und Waschmedium andererseits,
(b) stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei der Druckabfall pro Mischelement 0,1 bar bis 3,0 bar beträgt und durch die Mischelemente eine Mischenergie von 10 bis 1.000 Joule/Liter eingetragen wird, und
(c) stromabwärts zum Rohrreaktor angeordnet, eine Scheideeinrichtung (Scheider) zur Abtrennung der von den Verunreinigungen befreiten nitrierten Produkte vom Waschmedium.

Wie zuvor im Zusammenhang mit dem vorstehenden Verfahren geschildert, kann die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), sein.

Wie zuvor im Rahmen des vorstehenden Verfahrens beschrieben, kann die Dispergiereinrichtung, insbesondere das Mischorgan, dem Reaktor vorgeschaltet, insbesondere unmittelbar vorgeschaltet, sein. Insbesondere kann es in diesem Zusammenhang vorgesehen sein, dass die Dispergiereinrichung, insbesondere das Mischorgan, in den Rohrreaktor übergeht.

Gemäß einer alternativen Ausführungsform kann die Dispergiereinrichtung, insbesondere das Mischorgan, in den Rohrreaktor integriert sein und/oder Bestandteil des Rohrreaktors sein. Diesbezüglich kann auf die obigen Ausführungen im Zusammenhang mit dem vorstehenden Verfahren verwiesen werden.

Wie zuvor bei der Schilderung des vorstehenden Verfahrens erläutert, kann der Rohrreaktor mit Mischelementen, insbesondere zum Eintrag von zusätzlicher Mischenergie, ausgestattet sein. Wie zuvor beschrieben, können die Mischelemente als Bleche, insbesondere Prall- oder Umlenkbleche, als Blenden, als statische Mischer oder als Stromteiler ausgebildet ist.

Im Rahmen der erfindungsgemäßen Vorrichtung kann eine ein-, zwei- oder dreistufige Wäsche des rohen Nitrierproduktes durchgeführt werden (d. h. saure Wäsche und/oder basische Wäsche und/oder neutrale Wäsche).

Weiterhin ist es erfindungsgemäß vorgesehen, dass - stromabwärts zum Rohrreaktor angeordnet - eine Abtrenneinrichtung, nämlich eine Scheideeinrichtung (Scheider bzw. Settler und/oder dynamischer Scheider bzw. Zentrifugalseparator), zur Abtrennung der von den Verunreinigungen befreiten nitrierten Produkte vom Waschmedium angeordnet ist.

Des Weiteren besteht im Rahmen der erfindungsgemäßen Vorrichtung die Möglichkeit, dass - stromabwärts zum Rohrreaktor und stromaufwärts zur Abtrenneinrichtung (d. h. mit anderen Worten zwischen Rohrreaktor und Abtrenneinrichtung) - ein Rührkessel und/oder Rührreaktor angeordnet ist. Insbesondere wird auf diese Weise die Kontakt- und/oder Verweilzeit zwischen nitrierten Produkten einerseits und dem Waschmedium andererseits verlängert.

Für weitergehende Einzelheiten zu der erfindungsgemäßen Vorrichtung bzw. Anlage kann auf die obigen Ausführungen zu dem vorstehend geschilderten Verfahren verwiesen werden, welche in Bezug auf die erfindungsgemäße Vorrichtung bzw. Anlage entsprechend gelten.

Die erfindungsgemäße Vorrichtung bzw. Anlage kann in einer Produktionsanlage zur Nitrierung nitrierbarer aromatischer Verbindungen mit nachfolgender Aufreinigung der bei der Nitrierung entstehenden nitrierten Rohprodukte eingesetzt werden,
wobei die Produktionsanlage die folgenden Einheiten umfasst:
(a) eine Nitriereinheit zur Nitrierung aromatischer Verbindungen, insbesondere mit einem oder mehreren entsprechenden Reaktionsbehältern zur Durchführung der Nitrierreaktion(en);
(b) gegebenenfalls, in Produktionslinie stromabwärts zur Nitriereinheit angeordnet, mindestens eine Abtrenneinrichtung, insbesondere eine Scheideeinrichtung (Scheider), zur Abtrennung der Nitrierendsäure von den nitrierten Rohprodukten,
(c) in Produktionslinie stromabwärts zur Nitriereinheit und zur gegebenenfalls vorhandenen Abtrenneinrichtung angeordnet, mindestens eine Wascheinrichtung zur Durchführung einer Wäsche der nitrierten Rohprodukte, wobei die Wascheinrichtung umfasst:
   - mindestens eine Dispergiereinrichtung, insbesondere mindestens ein Mischorgan, zum Inkontaktbringen und Emulgieren der aufzureinigenden nitrierten Rohprodukte einerseits und dem Waschmedium andererseits und,
   - stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei der Druckabfall pro Mischelement 0,1 bar bis 3,0 bar beträgt und durch die Mischelemente eine Mischenergie von 10 bis 1.000 Joule/Liter eingetragen wird,
(d) gegebenenfalls, in Produktionslinie stromabwärts zur Wascheinrichtung angeordnet, einen Rührkessel, insbesondere zur Erhöhung der Kontakt- und/oder Verweilzeit zwischen nitrierten Produkten einerseits und Waschmedium andererseits,
(e) in Produktionslinie stromabwärts zur Wascheinheit und zum gegebenenfalls vorhandenen Rührkessel angeordnet, eine Abtrenneinrichtung,
   nämlich eine Scheideeinrichtung (Scheider), zur Abtrennung der von den Verunreinigungen befreiten nitrierten Produkte vom Waschmedium.

Mit anderen Worten ist bei der vorstehenden Produktionsanlage die zuvor beschriebene Vorrichtung bzw. Anlage zur Aufreinigung, d. h. zur Entfernung von Verunreinigungen, Bestandteil dieser Produktionsanlage, nämlich in Form der Wascheinheit bzw. Wascheinrichtung (c), der optionalen Wascheinrichtung (d) sowie der Abtrenneinrichtung (e).

Wie zuvor geschildert, kann auch bei der vorstehenden Produktionsanlage die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), sein.

Gemäß einer besonderen Ausgestaltung der vorstehenden Produktionsanlage kann - wie zuvor geschildert - die Dispergiereinrichtung, insbesondere das Mischorgan, dem Reaktor vorgeschaltet, insbesondere unmittelbar vorgeschaltet, sein. Insbesondere kann bei dieser Ausführungsform die Dispergiereinrichung, insbesondere das Mischorgan, in den Rohrreaktor übergehen.

Gleichermaßen kann es erfindungsgemäß vorgesehen sein, dass die Dispergiereinrichtung, insbesondere das Mischorgan, in den Rohrreaktor integriert ist und/oder Bestandteil des Rohrreaktors ist. Bezüglich dieser Ausführungsform kann - zur Vermeidung unnötiger Wiederholungen - auf die obigen Ausführungen verwiesen werden.

Wie zuvor im Zusammenhang mit dem vorstehenden Verfahren und im Zusammenhang mit der erfindungsgemäßen Vorrichtung bzw. Anlage zur Aufreinigung geschildert, kann der Rohrreaktor mit Mischelementen, insbesondere zum Eintrag von zusätzlicher Mischenergie, ausgestattet sein. Insbesondere können bei dieser Ausführungsform die Mischelemente als Bleche, insbesondere Prall- oder Umlenkbleche, als Blenden, als statische Mischer oder als Stromteiler ausgebildet sein.

Das vorstehende Verfahren eignet sich insbesondere zur Durchführung einer sauren Wäsche und/oder einer basischen Wäsche und/oder einer neutralen Wäsche von nitrierten Rohprodukte. Das vorstehende Verfahren kann also in allen drei vorgenannten Waschschritten einer Wascheinrichtung zum Einsatz kommen. Gleichermaßen ist es aber auch möglich, das vorstehende Verfahren nur für eine oder zwei Waschschritte zu verwenden, beispielsweise nur für eine saure Wäsche oder aber nur für eine basische Wäsche oder aber nur für eine neutrale Wäsche. In dieser Hinsicht ist das vorstehende Verfahren flexibel einsetzbar.

Für weitergehende Einzelheiten zu der vorstehenden Produktionsanlage kann auf die obigen Ausführungen zu dem vorstehenden Verfahren und zu der erfindungsgemäßen Vorrichtung bzw. Anlage verwiesen werden, welche in Bezug auf die vorstehende Produktionsanlage entsprechend gelten.

Das vorstehende Verfahren und die erfindungsgemäße Vorrichtung bzw. Anlage zur Aufreinigung sowie die vorstehende Produktionsanlage zur Nitrierung sind in den beigefügten Figurendarstellungen beispielhaft und in nichtbeschränkender Weise veranschaulicht.

Weitere Vorteile, Eigenschaften, Aspekte und Merkmale der vorliegenden Erfindung ergeben sich aus der folgenden Beschreibung von in den Zeichnungen dargestellten, erfindungsgemäß bevorzugten Ausführungsformen. Es zeigt:
- Fig. 1: eine schematische Darstellung einer Wäsche von Nitroaromaten nach dem Stand der Technik mittels Mixer/Settler-Technologie für die üblichen drei Waschstufen einer Wäsche von Nitroaromaten;
- Fig. 2: eine schematische Darstellung einer einstufigen Wäsche für Nitroaromaten nach dem vorstehenden Verfahren bzw. mit der erfindungsgemäßen Vorrichtung bzw. Anlage;
- Fig. 3: eine schematische Darstellung eines Ablaufs des vorstehenden Verfahrens bzw. eine schematische Darstellung der erfindungsgemäßen Vorrichtung bzw. Anlage gemäß einem bevorzugten Ausführungsbeispiel der Erfindung für die üblichen drei Waschstufen einer Wäsche von Nitroaromaten;
- Fig. 4: eine schematische Darstellung einer vorstehenden Produktionsanlage zur Nitrierung nitrierbarer aromatischer Verbindungen mit nachfolgender Wäsche der erhaltenen Nitroaromaten gemäß einem bevorzugten Ausführungsbeispiel der Erfindung.

Fig. 1 zeigt ein Beispiel für eine Wasche von Nitroaromaten in drei Schritten nach dem Stand der Technik:
a) In Schritt 1 werden in einer mehrstufigen kontinuierlichen sauren Wäsche (WS) die im rohen Nitroaromaten (NA 10) suspendierte und gelöste Schwefel- und Salpetersäure durch Wäsche mit Frischwasser (WW 10) ausgewaschen. Der zu waschende Nitroaromat (NA 10) und das Waschwasser (WW 10) werden in eine Mischeinrichtung, üblicherweise einem Rührkessel, mit einer Verweilzeit von ca. 10 Minuten. eingespeist. Die gebildete Waschemulsion wird anschließend in einem Scheider (S) getrennt. Zur vollständigen Entfernung der gelösten und suspendierten Mineralsäuren können bis zu 4 Mixer/Settler-Einheiten (n = 3) zum Einsatz kommen, wobei das Waschmedium und der zu waschende Nitroaromat im Gegenstrom geführt werden. Das Waschmedium wird, nach Phasentrennung, entweder als Abwasser (WW 11) sofort vollständig abgegeben, oder eine Teilmenge wird zusätzlich im Kreis geführt, um ein vorgegebenes Phasenverhältnis und damit einen definierten Emulsionstyp einzustellen und um die Zeit zur Trennung der Phasen zu minimieren. Der von Mineralsäuren befreite Nitroaromat (NA 11) wird in Waschstufe 2, die alkalische Wäsche (WA), eingespeist.
b) In Schritt 2 werden in einer mehrstufigen kontinuierlichen alkalischen Wäsche (WA) alle gelösten Nitrophenole, Nitrobenzoesäuren und andere aciden Stoffe aus dem oxidativen Abbau von Verunreinigungen und isomere Nitroaromaten aus dem Nitroaromaten (z. B. TNT) entfernt. Der zu waschende Nitroaromat (NA 11) und das Waschwasser (WW 10 bzw. WW13) werden, zusammen mit einer Base, in einen Rührkessel mit einer Verweilzeit von ca. 10 Minuten. eingespeist. Die gebildete Waschemulsion wird anschließend in einem Scheider (S) getrennt. Zur vollständigen Entfernung der im Nitroaromat gelösten Nitrophenole, Nitrobenzoesäuren und anderen aciden Stoffen aus dem oxidativen Abbau von Verunreinigungen und isomeren Nitroaromaten können bis zu 4 Mixer/Settler-Einheiten (n = 3) zum Einsatz kommen, wobei das Waschmedium und der zu waschende Nitroaromat im Gegenstrom geführt werden. Das Waschmedium wird, nach Phasentrennung, entweder als Abwasser (WW 12) vollständig sofort abgegeben, oder eine Teilmenge wird zusätzlich noch im Kreis geführt, um ein vorgegebenes Phasenverhältnis und damit einen definierten Emulsionstyp einzustellen und um die Zeit zu Trennung der Phasen zu minimieren. Der von Mineralsäuren, Nitrophenolen, Nitrobenzoesäuren und anderen aciden Stoffe aus dem oxidativen Abbau von Verunreinigungen und isomeren Nitroaromaten befreite Nitroaromat (NA 12) wird in Waschstufe 3, die Neutralwäsche (WN), eingespeist.
c) In Schritt 3 werden in einer mehrstufigen Neutralwäsche (WN) die mitgerissenen Spuren an Waschmedium aus der alkalischen Wäsche (WA) entfernt. Der zu waschende Nitroaromat (NA 12) und das Waschwasser (WW 10) werden in einen Rührkessel mit einer Verweilzeit von ca. 10 Minuten. eingespeist. Die gebildete Waschemulsion wird anschließend in einem Scheider (S) getrennt. Zur vollständigen Entfernung der im Nitroaromat noch suspendierten bzw. gelösten Spuren an Base können bis zu 4 Mixer/Settler-Einheiten (n = 3) zum Einsatz kommen, wobei das Waschmedium und der zu waschende Nitroaromat im Gegenstrom geführt werden. Die wässrige Phase wird entweder als Waschmedium (WW 13) sofort vollständig in die alkalische Wäsche (WA) eingespeist, oder eine Teilmenge wird zusätzlich im Kreis geführt, um ein vorgegebenes Phasenverhältnis und damit einen definierten Emulsionstyp einzustellen und um die Zeit zu Trennung der Phasen zu minimieren. Der jetzt von Mineralsäuren, Nitrophenolen, Nitrobenzoesäuren und anderen aciden Stoffe aus dem oxidativen Abbau von Verunreinigungen, isomeren Nitroaromaten und Restspuren Alkali befreite Nitroaromat (NA 13) wird direkt zur Weiterverarbeitung oder in ein Zwischenlager abgegeben.

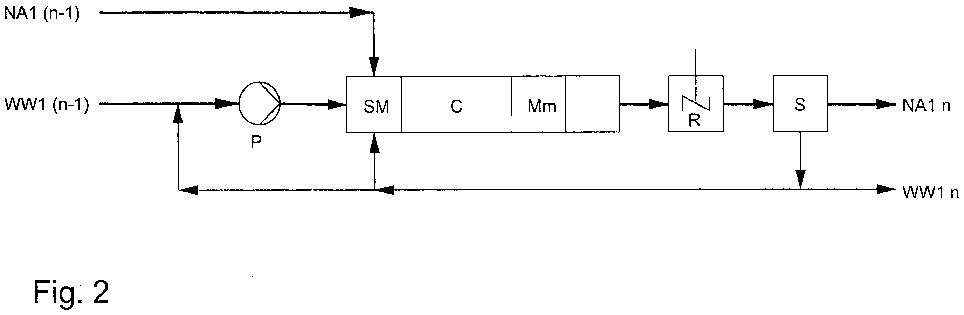

Fig. 2 zeigt eine Ausführungsform für eine Waschstufe nach dem vorstehenden Verfahren bzw. mit der erfindungsgemäßen Vorrichtung bzw. Anlage zur Wäsche von Nitroaromaten mit dem Waschmedium als Treibstrahl.

Der zu waschende Nitroaromat, nach der Abtrennung der Nitrierendsäure (NA1 (n-1) mit n = 1)) oder nach der Entfernung der im Nitroaromaten als Mikroemulsion noch suspendierten Nitrierendsäure oder der im Nitroaromaten noch gelösten Schwefelsäure, Salpetersäure und Nitrose in einer sauren Wäsche (WS mit n = 2) oder nach der Entfernung aller im Nitroaromaten gelösten Nitrophenole, Nitrobenzoesäuren und anderen aciden Stoffen aus dem oxidativen Abbau von Verunreinigungen und isomerer Nitroaromaten aus dem Nitroaromaten (z.B. TNT) in Gegenwart von Basen in einer alkalische Wäsche (WA mit n = 3) wird in einem Strahlmischer (SM) mit dem Waschmedium WW1 (n-1), das im dargestellten Fall als Treibstrahl dient, zusammengebracht und direkt in einen Rohrreaktor (C) der zusätzliche Mischelementen (Mm) enthält, eingebracht.

Zur Einstellung einer verlängerten Verweilzeit, um langsam ablaufende Umsetzungen der auszuwaschenden Verunreinigungen im Waschmedium, wie Nitrose, zuzulassen, kann die Waschemulsion aus dem Rohrreaktor in einen Verweilzeitbehälter, wie z. B. einen oder mehrere Rührkessel (R), eingespeist werden. Die Waschemulsion aus dem Rohrreaktor wird direkt oder nach einer verlängerten Verweilzeit im Rührkessel in einer Scheideeinrichtung in die Phasen getrennt.

Der gewaschene Nitroaromat (NA1 n mit n = 1 bis 3) wird entweder in die nachfolgende Waschstufe bzw. als fertig gewaschenes Produkt (NA13) zur Weiterverarbeitung abgegeben. Das beladene Waschmedium (WW1 n mit n = 1 bis 3) wird entweder direkt als Abwasser abgegeben oder als Teilstrom zur Einstellung eines definierten Phasenverhältnisses zwischen Nitroaromat und Waschmedium zurückgeführt. Dieser zurückgeführte Teilstrom kann entweder zusammen mit dem neu zugesetzten Waschwasser als Treibstrahl oder als Umlaufstrom direkt in den Rohrreaktor eingespeist werden.

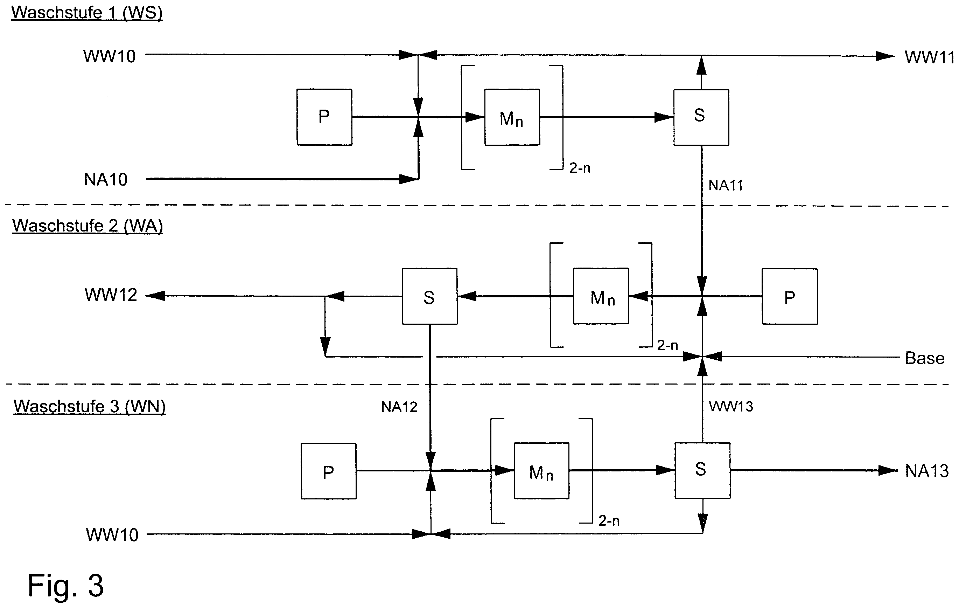

Fig. 3 zeigt ein Beispiel des vorstehenden Verfahrens in drei Schritten für die separate Entfernung der Mineralsäuren mittels saurer Wäsche (WS), die Entfernung aller gelöster Nitrophenole, Nitrobenzoesäuren und anderer acider Stoffe aus dem oxidativen Abbau von Verunreinigungen und isomerer Nitroaromaten in Gegenwart von Basen im alkalischen Bereich mittels alkalischer Wäsche (WA) und eine Neutralwäsche (WN).
a) In Schritt 1 werden in einer einstufigen sauren Wäsche (WS) die im rohen Nitroaromaten (NA 10) suspendierte und gelöste Schwefel- und Salpetersäure durch Wäsche mit Frischwasser (WW 10) entfernt. Der zu waschende Nitroaromat (NA 10) und das Waschwasser (WW 10) werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider (S) getrennt. Das Waschmedium wird, nach Phasentrennung, entweder als Abwasser (WW 11) direkt abgegeben, oder eine Teilmenge wird zusätzlich im Kreis geführt für die Einstellung eines vorgegebenen Phasenverhältnisses und damit eines definierten Emulsionstyps und um die Zeit zur Trennung der Phasen zu minimieren. Der von Mineralsäuren befreite Nitroaromat (NA 11) wird in Waschstufe 2, die alkalische Wäsche (WA), eingespeist.
b) In Schritt 2 werden in einer einstufigen alkalischen Wäsche (WA) alle gelösten Nitrophenole, Nitrobenzoesäuren und anderen aciden Stoffe aus dem oxidativen Abbau von Verunreinigungen und isomeren Nitroaromaten aus dem Nitroaromaten entfernt. Der zu waschende Nitroaromat (NA 11) nach der sauren Wäsche (WS) und das Waschwasser (WW 10 bzw. WW13 aus der Neutralwäsche) und eine Base werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider getrennt. Das Waschmedium, das alle gelösten Nitrophenole, Nitrobenzoesäuren und andere acide Stoffe aus dem oxidativen Abbau von Verunreinigungen und extrahierte isomere Nitroaromaten (als Salz gelöst) enthält, wird nach Phasentrennung entweder als Abwasser (WW 12) direkt abgegeben, oder eine Teilmenge wird im Kreis geführt zur Einstellung eines vorgegebenen Phasenverhältnisses und damit eines definierten Emulsionstyps und um die Zeit zur Trennung der Phasen zu minimieren. Der von Mineralsäuren, Nitrophenolen, Nitrobenzoesäuren und andere aciden Stoffen aus dem oxidativen Abbau von Verunreinigungen und isomeren Nitroaromaten befreite Nitroaromat (NA 12) wird in Waschstufe 3, die Neutralwäsche (WN), eingespeist.
c) In Schritt 3 werden in einer einstufigen Neutralwäsche (WN) die mitgerissenen Spuren an Waschmedium aus der alkalischen Wäsche entfernt. Der zu waschende Nitroaromat (NA 12) und das Waschwasser (WW 10) werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider (S) getrennt. Das Waschmedium, das die Restspuren an Alkali und Verunreinigungen enthält, wird entweder als Abwasser (WW 13) direkt in die Waschstufe 2 (WA) eingeführt, oder eine Teilmenge wird zusätzlich im Kreis geführt zur Einstellung eines vorgegebenen Phasenverhältnisses und damit eines definierten Emulsionstyps und um die Zeit zur Trennung der Phasen zu minimieren. Der jetzt von Mineralsäuren, Nitrophenolen, Nitrobenzoesäuren und andere aciden Stoffen aus dem oxidativen Abbau von Verunreinigungen, isomeren Nitroaromaten und Restspuren Alkali befreite Nitroaromat (NA 13) wird direkt zur Weiterverarbeitung oder in ein Zwischenlager abgegeben.

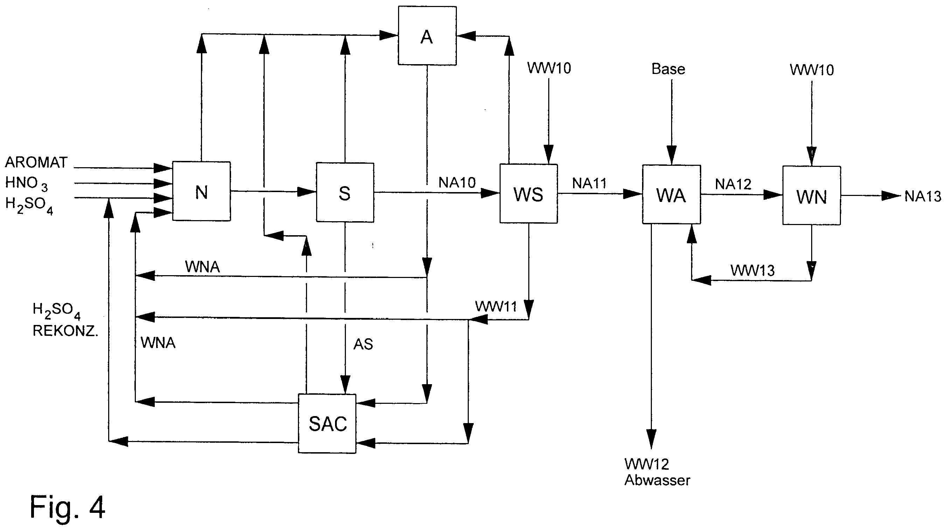

Fig. 4 zeigt ein Beispiel für eine Produktionsanlage zur Herstellung von Nitroaromaten mit integrierter erfindungsgemäßer Wäsche der rohen Nitroaromaten aus einer isothermen oder adiabatischen Nitrierung. Der in der Nitriereinheit (N) durch Umsetzung des Aromaten mit Salpetersäure in Gegenwart von Schwefelsäure gebildete rohe Nitroaromat (NA 10) wird nach Abtrennung der Nitriersäure im Scheider (S) in der sauren Wäsche (WS) mit Wasser (WW 10) in der erfindungsgemäßen Weise gewaschen. Nach Phasentrennung wird das resultierende Abwasser (WW 11), das alle ausgewaschene Schwefel- und Salpetersäure enthält, zusammen mit der aus der Abgasbehandlung der Nitrieranlage in einer Absorberanlage (A) erhaltenen Salpetersäure (WNA), entweder direkt oder nach Aufkonzentrierung in einer SAC-Anlage (SAC), zusammen mit der Endsäure (AS) aus der Nitrierung wieder in die Nitrierung zurückgeführt oder als zu behandelndes Abwasser abgegeben.

Der von den Mineralsäuren befreite Nitroaromat (NA 11) wird in der Waschstufe 2 (alkalische Wäsche WA) in Gegenwart von Basen nach dem vorstehenden Verfahren quasi einstufig gewaschen. Nach Phasentrennung wird das Abwasser aus der alkalischen Wäsche (WW12) mit einem pH-Wert im Bereich von 8,0 bis 13, das alle Nitrophenole, Nitrobenzoesäuren und anderen aciden Stoffe aus dem oxidativen Abbau von Verunreinigungen und isomeren Nitroaromaten (z. B. TNT) enthält, vor Abgabe in einen Vorfluter einer zusätzlichen Behandlung zugeführt, wie z. B. einer Thermolyse.

Der Nitroaromat (NA 12) aus der alkalischen Wäsche (WA) wird in die Neutralwäsche (WN) eingespeist und mit Wasser (WW 10) nach dem vorstehenden Verfahren quasi einstufig gewaschen. Nach Phasentrennung wird das Abwasser (WW 13) aus der Neutralwäsche (WN) in die Waschstufe 2 (WA) zusammen mit Base eingespeist. Der gewaschene Nitroaromat (NA 13) wird in die Weiterverarbeitung, wie z. B. in eine Isomerentrennung, die Reduktion zu dem entsprechenden Amin oder in ein Zwischenlager, abgegeben.

Weitere Ausgestaltungen, Abwandlungen, Variationen der vorliegenden Erfindung sind für den Fachmann beim Lesen der Beschreibung ohne Weiteres erkennbar und realisierbar, ohne dass der Rahmen der vorliegenden Erfindung verlassen wird.

Die vorliegende Erfindung wird anhand der folgenden Ausführungsbeispiele veranschaulicht, ohne jedoch die vorliegende Erfindung hierauf zu beschränken.

Auch wenn in den nachfolgenden Ausführungsbeispielen das vorstehende Verfahren bzw. die erfindungsgemäße Vorrichtung mit Nitrobenzol als aufzureinigendem Nitroaromaten veranschaulicht wird, ist das Verfahren bzw. die Vorrichtung nach der vorliegenden Erfindung keinesfalls darauf beschränkt, sondern ist auch auf beliebige andere Nitroaromaten, z. B. aus der Nitrierung von Toluol, Chlorbenzolen, Xylolen, Nitrobenzolen etc., und auf beliebige andere Basen als Natronlauge übertragbar.

### Ausführungsbeispiele:

### Beispiel 1: Einstufige alkalische Wäsche (Vergleichsbeispiel)

12 kg/h eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6-Dinitrophenol (2,6-DNP) und 360 ppm an Pikrinsäure (2,4,6-TNP)), wurde zusammen mit einer Waschlauge mit 0,8 g NaOH/l (zweifacher Überschuss, bezogen auf alle Nitrophenole) entsprechend einem Gewichtsverhältnis 1 : 1 in einen Rührkessel bei 60 °C eingespeist. Die Rührerdrehzahl wurde so eingestellt, dass im Rührkessel eine O/W-Emulsion mit dem dosierten Phasenverhältnis vorlag. Die Verweilzeit im Rührkessel betrug 6 Minuten. Nach Phasentrennung (ca. 40 Minuten) lag der pH-Wert in der Waschlauge, die 1.850 ppm an Nitrophenolen enthielt, bei ca. 11,7. Im gewaschenen Nitrobenzol wurden 60 ppm Nitrophenole gefunden. Bei Einsatz einer Waschlauge mit 4 g/l Natronlauge unter sonst gleichen Bedingungen konnte die Scheidezeit fast um den Faktor 4 auf ca. 15 Minuten verkürzt werden.

### Beispiel 2: Einstufige alkalische Wäsche (erfindungsgemäß)

12 kg/h eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6-Dinitrophenol (2,6-DNP) und 360 ppm an Pikrinsäure (2,4,6-TNP)), wurde mit einer Waschlauge mit 0,8 g NaOH/l (zweifacher Überschuss, bezogen auf alle Nitrophenole) im Gewichtsverhältnis 1 : 1 mittels eines Strahlmischers mit dem Waschmedium als Zentralstrahl bei 60 °C in einen Rohrreaktor eingespeist, der noch zusätzlich 5 statische Mischelemente enthielt. Die Relativgeschwindigkeit zwischen Zentralstrahl und zu waschendem Nitrobenzol betrug 8 : 1. Die Verweilzeit im Rohrreaktor betrug nicht mehr als 5 Sekunden. Der Druckabfall über die gesamte Rohrreaktorlänge betrug 1,6 bar. Nach Phasentrennung der O/W-Emulsion (ca. 40 Minuten) lag der pH-Wert in der Waschlauge, die 1.908 ppm an Nitrophenolen enthielt, bei ca. 11,6. Im gewaschenen Nitrobenzol wurden 2 ppm Nitrophenole gefunden. Bei Einsatz einer Waschlauge mit 4 g/l Natronlauge unter sonst gleichen Bedingungen konnte die Scheidezeit um den Faktor 4 auf ca. 10 Minuten verkürzt werden. Mit dem zu waschenden Nitroaromaten als Zentralstrahl im Strahlmischer wurden die gleichen Ergebnisse erzielt.

### Beispiel 3: Einstufige Neutralwäsche (erfindungsgemäß)

12 kg/h eines Nitrobenzols aus einer adiabatischen Nitrierung, das nach einer Wäsche mit Alkali (siehe z. B. Beispiel 2, alkalische Wäsche) und das noch insgesamt 2 bis 5 ppm an Nitrophenolen enthielt, wurde im Gewichtsverhältnis 1 : 1 mittels eines Strahlmischers mit Wasser als Zentralstrahl bei 60 °C in einen Rohrreaktor eingespeist, der noch zusätzlich 2 statische Mischelemente enthielt. Die Relativgeschwindigkeit zwischen Zentralstrahl und zu waschendem Nitrobenzol betrug 8 : 1. Die Verweilzeit im Rohrreaktor lag bei ca. 5 Sekunden. Der Druckabfall über die gesamte Rohrreaktorlänge betrug 0,6 bar. Nach Phasentrennung (ca. 25 Minuten) lag der pH-Wert im Waschwasser mit ca. 1,5 bis 4,5 ppm an Nitrophenolen bei ca. 9,0. Im gewaschenen Nitrobenzol wurden noch 0,5 ppm Nitrophenole gefunden. Mit dem zu waschenden Nitroaromaten als Zentralstrahl im Strahlmischer wurden die gleichen Ergebnisse erzielt.

### Beispiel 4: Einstufige alkalische Wäsche (erfindungsgemäß)

20 kg/h kg eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6 Dinitrophenol (2,6-DNP) und 360 ppm an Picrinsäure (2,4,6-TNP)), wurde mit 4 kg/h Waschlauge mit 4 g NaOH/l (zweifacher Überschuss, bezogen auf alle Nitrophenole) entsprechend einem Gewichtsverhältnis von Nitroaromat zu Waschlauge von 5 : 1 direkt gewaschen, wobei das Waschmedium mittels eines Strahlmischers und der zu waschende Nitroaromat bei 60 °C in einen Rohrreaktor eingespeist wurden, der noch zusätzlich 5 statische Mischelemente enthielt. Die Relativgeschwindigkeit zwischen Zentralstrahl und zu waschendem Nitrobenzol betrug 8 : 1. Die Verweilzeit im Rohrreaktor betrug nicht mehr als 5 Sekunden. Der Druckabfall über die gesamte Rohrreaktorlänge betrug 1,6 bar. Nach Phasentrennung der Emulsion vom Typ W/O (ca. 5 Minuten) lag der pH-Wert in der Waschlauge, die 9.552 ppm an Nitrophenolen enthielt, bei ca. 12,3. Im gewaschenen, noch trüben Nitrobenzol wurden ca. 8 ppm Nitrophenole gefunden.

### Beispiel 5: Einstufige Neutralwäsche (erfindungsgemäß)

20 kg/h eines Nitrobenzols aus einer adiabatischen Nitrierung, das nach einer Wäsche mit Alkali (siehe Beispiel 2, alkalische Wäsche) insgesamt noch 5 bis 8 ppm an Nitrophenolen enthielt, wurde im Gewichtsverhältnis 5 : 1 mittels eines Strahlmischers mit Wasser als Zentralstrahl bei 60 °C in einen Rohrreaktor eingespeist, der noch zusätzlich 2 statische Mischelemente enthielt. Die Relativgeschwindigkeit zwischen Zentralstrahl und zu waschendem Nitrobenzol betrug 8 : 1. Die Verweilzeit im Rohrreaktor lag bei ca. 5 Sekunden. Der Druckabfall über die gesamte Rohrreaktorlänge betrug 0,6 bar. Nach Phasentrennung (ca. 20 Minuten) lag der pH-Wert im Waschwasser mit ca. 1,5 bis 4,5 ppm an Nitrophenolen bei ca. 9,0. Im gewaschenen Nitrobenzol wurden noch 0,5 ppm Nitrophenole gefunden. Mit dem zu waschenden Nitroaromaten als Zentralstrahl im Strahlmischer wurden die gleichen Ergebnisse erzielt.

## Patentansprüche

1. Vorrichtung (Anlage) zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium,
wobei die Vorrichtung die folgenden Einrichtungen aufweist:
(a) mindestens eine Dispergiereinrichtung, insbesondere mindestens ein Mischorgan, zum Inkontaktbringen und Emulgieren von aufzureinigenden nitrierten Rohprodukten einerseits und Waschmedium andererseits;
(b) stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder neutralisiert werden, wobei der Rohrreaktor mit Mischelementen zum Eintrag von zusätzlicher Mischenergie ausgestattet ist, wobei der Druckabfall pro Mischelement 0,1 bar bis 3,0 bar beträgt und durch die Mischelemente eine Mischenergie von 10 bis 1000 Joule/Liter eingetragen wird; und
(c) stromabwärts zum Rohrreaktor angeordnet, eine Scheideeinrichtung (Scheider) zur Abtrennung der von den Verunreinigungen befreiten nitrierten Produkte vom Waschmedium.

2. Vorrichtung nach Anspruch 1, **dadurch gekennzeichnet, dass** die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), ist.

3. Vorrichtung nach Anspruch 1 oder 2, **dadurch gekennzeichnet, dass** die Dispergiereinrichtung, insbesondere das Mischorgan, dem Reaktor vorgeschaltet, insbesondere unmittelbar vorgeschaltet, ist, insbesondere wobei die Dispergiereinrichung, insbesondere das Mischorgan, in den Rohrreaktor übergeht.

4. Vorrichtung nach Anspruch 1 oder 2, **dadurch gekennzeichnet, dass** die Dispergiereinrichtung, insbesondere das Mischorgan, in den Rohrreaktor integriert ist und/oder Bestandteil des Rohrreaktors ist.

5. Vorrichtung nach Anspruch 2, **dadurch gekennzeichnet, dass** der Strahlmischer einen vorzugsweise zentralen Treibstahl und einen den Treibstrahl umgebendes Medium, insbesondere in Form eines Ringstrahls, erzeugt.

6. Vorrichtung nach einem der vorangehenden Ansprüche, **dadurch gekennzeichnet, dass** die Mischelemente als Bleche, insbesondere Prall- oder Umlenkbleche, als Blenden, als statische Mischer oder als Stromteiler ausgebildet sind.

7. Vorrichtung nach einem der vorangehenden Ansprüche, **dadurch gekennzeichnet, dass** der Druckabfall pro Mischelement 0,3 bis 1,5 bar, besonders bevorzugt 0,3 bis 0,8 bar, beträgt und dass durch die Mischelemente eine Mischenergie von 10 bis 500 Joule/Liter, besonders bevorzugt 20 bis 200 Joule/Liter, eingetragen wird.

8. Vorrichtung nach einem der vorangehenden Ansprüche, **dadurch gekennzeichnet,**
**dass**, stromabwärts zum Rohrreaktor und stromaufwärts zur Abtrenneinrichtung und/oder zwischen Rohrreaktor und Abtrenneinrichtung, ein Rührkessel angeordnet ist, insbesondere zur Erhöhung der Kontakt- und/oder Verweilzeit zwischen nitrierten Produkten einerseits und Waschmedium andererseits.

9. Verwendung einer Vorrichtung gemäß einem der Ansprüche 1 bis 8 zur Durchführung eines Verfahrens zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium,
wobei bei dem Verfahren
(a) zunächst die nitrierten Rohprodukte mit einem Waschmedium in Kontakt gebracht werden und die nitrierten Rohprodukte und das Waschmedium ineinander verteilt werden derart, dass eine Emulsion resultiert,
(b) nachfolgend die resultierende Emulsion in einen Rohrreaktor einspeist wird, so dass während des Durchtritts der Emulsion durch den Rohrreaktor die in den nitrierten Rohprodukten anfänglich vorhandenen Verunreinigungen entfernt werden und/oder so dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, und
(c) sich Verfahrensschritt (b) eine Abtrennung der von den Verunreinigungen befreiten nitrierten Produkte vom Waschmedium in einer Scheideeinrichtung (Scheider) anschließt.

10. Verwendung nach Anspruch 9, **dadurch gekennzeichnet, dass** als Dispergiereinrichtung, insbesondere als Mischorgan, ein Strahlmischer (Jet-Mixer) eingesetzt wird, insbesondere wobei der Strahlmischer einen vorzugsweise zentralen Treibstahl und einen den Treibstrahl umgebendes Medium, insbesondere in Form eines Ringstrahls, erzeugt.

11. Verwendung nach Anspruch 10, **dadurch gekennzeichnet, dass** der Treibstrahl im Strahlmischer das Waschmedium oder aber das aufzureinigende nitrierte Rohprodukt ist und/oder dass das Verhältnis der Geschwindigkeiten zwischen dem zentralen Treibstrahl einerseits und dem den zentralen Treibstrahl umgebenden Medium, insbesondere Ringstrahl, im Strahlmischer im Bereich von 1 : 5 bis 30 : 1, bevorzugt im Bereich von 1 : 2 bis 20 : 1, besonders bevorzugt im Bereich von 1 : 1 bis 10 : 1, eingestellt wird.

12. Verwendung nach einem der vorangehenden Ansprüche, **dadurch gekennzeichnet,**
**dass** die Verweilzeit im Rohrreaktor 0,1 bis 120 Sekunden, bevorzugt 0,1 bis 60 Sekunden, besonders bevorzugt 1 bis 30 Sekunden, beträgt.

13. Verwendung nach einem der vorangehenden Ansprüche, **dadurch gekennzeichnet,**
**dass** das Masseverhältnis zwischen aufzureinigenden nitrierten Rohprodukten einerseits und Waschmedium andererseits im Bereich von 200 : 1 bis 1 : 10, bevorzugt im Bereich von 100 : 1 bis 1 : 5, besonders bevorzugt im Bereich von 10:1 bis 1 : 2, eingestellt und/oder dass das Phasenverhältnis zwischen aufzureinigenden nitrierten Rohprodukten einerseits und Waschmedium andererseits im Bereich von 25 : 1 bis 1 : 5, insbesondere im Bereich von 10 : 1 bis 1 : 2, bevorzugt im Bereich von 5 : 1 bis 1 : 1, eingestellt wird.

14. Verwendung nach einem der vorangehenden Ansprüche, **dadurch gekennzeichnet,**
**dass** das Waschmedium unter Verfahrensbedingungen, insbesondere bei Temperaturen ab 5 °C, insbesondere bei Temperaturen ab 25 °C, und Atmosphärendruck, flüssig ist und bevorzugt wässrig basiert ist, vorzugsweise Wasser ist und/oder
**dass** die aufzureinigenden nitrierten Rohprodukte unter Verfahrensbedingungen, insbesondere bei Temperaturen ab 5 °C, insbesondere bei Temperaturen ab 25 °C, und Atmosphärendruck, flüssig sind und/oder dass die aufzureinigenden nitrierten Rohprodukte aus der Nitrierung von ein- oder mehrkernigen Aromaten, insbesondere Benzol, Toluol, Xylol oder halogenierten Aromaten, insbesondere chlorierten Benzolen, stammen und/oder
**dass** die aufzureinigenden nitrierten Rohprodukte gegebenenfalls halogenierte Mono-, Di- und Trinitroaromaten sind.

## Claims

1. Apparatus (plant) for removing impurities from nitrided raw products obtained during the nitration of nitratable aromatic compounds after separation of the nitrating end acid by treatment with a scrubbing medium,
wherein the apparatus comprises the following devices:
a) at least one dispersing device, in particular at least one mixing device, for bringing into contact and emulsifying nitrided raw products to be purified, on the one hand, and scrubbing medium, on the other;
b) a tube reactor is arranged downstream of the dispersing device to feed the emulsion of nitrided raw products to be purified in the dispersing device, on the one hand, and scrubbing medium on the other, wherein the tube reactor is so designed that during the passage of the emulsion through the tube reactor, removal of the impurities originally present in the nitrided raw products is made possible and/or during the passage of the emulsion through the tube reactor, the impurities originally present in the nitrided raw products are transferred into the scrubbing medium and/or neutralized, wherei the tube reactor is equipped with mixing devices, in particular for the input of additional mixing energy, wherein the pressure drop per mixing device is 0.1 bar to 3.0 bar and wherein a mixing energy of 10 to 1000 joules/liter is introduced through the mixing devices; and
c) a separation device (separator) is arranged downstream of the tube reactor to separate the nitrided products freed of impurities from the scrubbing medium.

2. Apparatus according to claim 1,
**characterized in that** the dispersing device, in particular the mixing device, is a stirrer tank, a jet mixer or a pump, in particular a centrifugal pump, preferably a pump, in particular a centrifugal pump or a jet mixer, particularly preferably a jet mixer.

3. Apparatus according to claim 1 or 2,
**characterized in that** the dispersing device, in particular the mixing device, is connected upstream of the tube reactor, in particular directly upstream, in particular wherein the dispersing device, in particular the mixing device, merges into the tube reactor.

4. Apparatus according to claim 1 or 2,
**characterized in that** the dispersing device, in particular the mixing device, is integrated into the tube reactor and/or is a component of the tube reactor.

5. Apparatus according to claim 2,
**characterized in that** the jet mixer generates a preferably central drive jet and a medium surrounding the drive jet, in particular in the form of a ring jet

6. Apparatus according to one of the preceding claims,
**characterized in that** the mixing devices are in the form of plates, in particular baffle plates or deflection plates, diaphragms, static mixers or flow dividers.

7. Apparatus according to one of the preceding claims,
**characterized in that** the pressure drop per mixing device is 0.3 to 1.5 bar, particularly preferably 0.3 to 0.8 bar, and that a mixing energy of 10 to 500 joules/liter, particularly preferably 20 to 200 joules/liter, is introduced through the mixing devices.

8. Apparatus according to one of the preceding claims,
**characterized in that** a stirrer tank is arranged downstream of the tube reactor and upstream of the separation device and/or between the tube reactor and the separation device, in particular for increasing the contact time and/or residence time between the nitrided products, on the one hand, and the scrubbing medium on the other.

9. Use of an apparatus according to one of the claims 1 to 8 to carry out a method for the removal of impurities from nitrated raw products obtained during the nitration of nitratable aromatic compounds after removal of the nitrating end acid by treatment with a scrubbing medium
a) first the nitrided raw products are brought into contact with a scrubbing medium and the nitrided raw products and the scrubbing medium are distributed into each other so that an emulsion results,
b) subsequently, the resulting emulsion is introduced into a tube reactor so that the impurities initially present in the nitrided raw products are removed during the passage of the emulsion through the tube reactor and/or so that during the passage of the emulsion through the tube reactor, the impurities initially present in the raw products are transferred into the scrubbing medium and/or neutralized thereby, and
c) following method step (b), separation of the nitrided products freed from impurities from the scrubbing medium in a separation device (separator).

10. Use according to claim 9,
**characterized in that** a jet mixer is used as a dispersing device in particular as a mixing device, in particular wherein the jet mixer generates a preferably central drive jet and a medium surrounding the drive jet, in particular in the form of a ring jet .

11. Use according to claim 10,
**characterized in that** the drive jet in the jet mixer is the scrubbing medium or the nitrided raw product to be purified and/or the ratio of the speeds between the central drive jet and the medium surrounding the central jet stream, in particular a ring jet, is adjusted in the range from 1: 5 to 30 : 1, preferably in the range from 1 : 2 to 20 : 1, particularly preferably in the range from 1 : 1 to 10 : 1.

12. Use according to one of the preceding claims,
**characterized in that** the residence time in the tube reactor is 0.1 to 120 seconds, preferably 0.1 to 60 seconds, particularly preferably 1 to 30 seconds.

13. Use according to one of the preceding claims,
**characterized in that** the ratio of the nitrided raw products to be purified, on the one hand, and the scrubbing medium, on the other, is in the range from 200 : 1 to 1 :10, preferably in the range from 100 : 1 to 1 : 5, particularly preferably in the range from 10 : 1 to 1 : 2, and/or the phase ratio of the nitrided raw products to be purified, on the one hand, and the scrubbing medium, on the other hand, is in the range from 25 : 1 to 1 : 5, in particular in the range from 10 : 1 to 1 : 2, preferably in the range from 10 : 1 to 1 : 2 more preferably in the range from 5 : 1 to 1 : 1.

14. Use according to one of the preceding claims,
**characterized in that** the scrubbing medium is liquid under method conditions, in particular at temperatures above 5 °C, in particular at temperatures above 25 °C and at atmospheric pressure, and is preferably aqueous-based, preferably water, and/or that the nitrided raw products to be purified are liquid under method conditions, in particular at temperatures above 5 °C, in particular at temperatures above 25 °C, and at atmospheric pressure, and/or the nitrided raw products to be purified are selected from the nitration of mono- or polyaromatic aromatics, in particular benzene, toluene, xylene or halogenated aromatics, in particular chlorinated benzenes, and/or the nitrided raw products to be purified are, if appropriate, halogenated mono-, di- and trinitroaromatics.

## Revendications

1. Dispositif (installation) pour éliminer des impuretés à partir de produits bruts nitrés, obtenus lors de la nitration de composés aromatiques pouvant être nitrés, après séparation du mélange sulfonitrique, par traitement avec un milieu de lavage,
le dispositif comprenant les équipements suivants :
(a) au moins un équipement disperseur, en particulier au moins un organe mélangeur, pour mise en contact et émulsification, d'une part de produits bruts nitrés à purifier, et d'autre part du milieu de lavage ;
(b) disposé en aval de l'équipement disperseur, un réacteur tubulaire, pour l'injection de l'émulsion, produite dans l'équipement disperseur, d'une part de produits bruts nitrés à purifier, et d'autre part du milieu de lavage, le réacteur tubulaire étant configuré de telle sorte que, pendant que l'émulsion traverse le réacteur tubulaire, une élimination soit possible des impuretés présentes initialement dans les produits bruts nitrés, et/ou que, pendant que l'émulsion traverse le réacteur tubulaire, les impuretés présentes initialement dans les produits bruts nitrés soient transférées dans le milieu de lavage et/ou neutralisées, où le réacteur tubulaire est muni d'éléments mélangeurs pour amener une énergie de mélange supplémentaire, où la perte de charge par élément mélangeur est de 0,1 bar à 0,3 bar et où une énergie de mélange de 10 à 1000 joules/litre est amenée aux éléments mélangeurs, et
(c) disposé en aval du réacteur tubulaire, un équipement séparateur (séparateur) pour séparer du milieu de lavage les produits nitrés débarrassés des impuretés.

2. Dispositif selon la revendication 1,
**caractérisé en ce que** l'équipement disperseur, en particulier l'organe mélangeur, est une cuve munie d'un agitateur, un mélangeur à jet (Jet-Mixer) ou une pompe, en particulier une pompe centrifuge, de préférence une pompe, en particulier une pompe centrifuge, ou un mélangeur à jet (Jet-Mixer), d'une manière particulièrement préférée un mélangeur à jet (Jet-Mixer).

3. Dispositif selon la revendication 1 ou 2,
**caractérisé en ce que** l'équipement disperseur, en particulier l'organe mélangeur, est installé en amont du réacteur, en particulier immédiatement en amont, en particulier dans lequel l'équipement disperseur, en particulier l'organe mélangeur, se poursuit à l'intérieur du réacteur tubulaire.

4. Dispositif selon la revendication 1 ou 2,
**caractérisé en ce que** l'équipement disperseur, en particulier l'organe mélangeur, est intégré dans le réacteur tubulaire et/ou est un constituant du réacteur tubulaire.

5. Dispositif selon la revendication 2,
**caractérisé en ce que** le mélangeur à jet produit un jet de propulsion, de préférence central, et un milieu entourant le jet de propulsion, en particulier sous forme d'un jet annulaire.

6. Dispositif selon l'une des revendications précédentes,
**caractérisé en ce que** les éléments mélangeurs sont configurés sous forme de tôles, en particulier de tôles de chicane et de déflecteurs, d'obturateurs, de mélangeurs statiques ou de diviseurs de courant.

7. Dispositif selon l'une des revendications précédentes,
**caractérisé en ce que** la perte de charge par élément mélangeur est de 0,3 à 1,5 bar, en particulier de 0,3 à 0,8 bar, et **en ce qu'**une énergie de mélange de 10 à 500 joules/litre, d'une manière particulièrement préférée de 20 à 200 joules/litre, est amenée aux éléments mélangeurs.

8. Dispositif selon l'une des revendications précédentes,
caractérisé, en aval du réacteur tubulaire et en amont de l'équipement séparateur et/ou entre le réacteur tubulaire et l'équipement séparateur, une cuve munie d'un agitateur est disposée, en particulier pour augmenter le temps de contact et/ou le temps de séjour entre d'une part les produits nitrés, et d'autre part le milieu de lavage.

9. Utilisation d'un dispositif selon l'une des revendications 1 à 8 pour la mise en oeuvre d'un procédé pour éliminer des impuretés de produits bruts nitrés, obtenus lors de la nitration de composés aromatiques pour être nitrés, après séparation du mélange sulfonitrique, par traitement avec un milieu de lavage, procédé dans lequel
(a) on met d'abord en contact les produits bruts nitrés avec un milieu de lavage, et on répartit les uns dans l'autre les produits bruts nitrés et le milieu de lavage de telle sorte qu'il en résulte une émulsion,
(b) puis on injecte dans un réacteur tubulaire l'émulsion obtenue, de telle sorte que, pendant le passage de l'émulsion à travers le réacteur tubulaire, il y ait élimination des impuretés présentes initialement dans les produits bruts nitrés, et/ou de telle sorte que, pendant le passage de l'émulsion à travers le réacteur tubulaire, il y ait transfert dans le milieu de lavage des impuretés présentes initialement dans les produits bruts nitrés, et/ou qu'il en résulte une neutralisation, et
(c) l'étape (b) est suivie d'une séparation, d'avec le milieu de lavage, des produits nitrés débarrassés des impuretés, dans un dispositif séparateur (séparateur).

10. Utilisation selon la revendication 9, **caractérisée en ce qu'**on utilise en tant qu'équipement disperseur, en particulier en tant qu'organe mélangeur, un mélangeur à jet (Jet-Mixer), en particulier pour laquelle le mélangeur à jet produit un jet de propulsion de préférence central et un milieu entourant le jet de propulsion, en particulier sous forme d'un jet annulaire.

11. Utilisation selon la revendication 10, **caractérisée en ce que** le jet de propulsion est, dans le mélangeur à jet, le milieu de lavage ou cependant le produit brut nitré à purifier, et/ou que le rapport des vitesses entre d'une part le jet de propulsion central et le milieu entourant le jet de propulsion central, en particulier le jet annulaire, est ajusté dans le mélangeur à jet dans la plage de 1:5 à 30:1, de préférence dans la plage de 1:2 à 20:1, d'une manière particulièrement préférée dans la plage de 1:1 à 10:1.

12. Utilisation selon l'une des revendications précédentes, **caractérisée en ce que** le temps de séjour dans le réacteur tubulaire est de 0,1 à 120 secondes, de préférence de 0,1 à 60 secondes, d'une manière particulièrement préférée de 1 à 30 secondes.

13. Utilisation selon l'une des revendications précédentes, **caractérisée en ce que** le rapport en masse entre d'une part les produits bruts nitrés à purifier et d'autre part le milieu de lavage est ajusté à une valeur comprise dans la plage de 200:1 à 1:10, de préférence dans la plage de 100:1 à 1:5, d'une manière particulièrement préférée dans la plage de 10:1 à 1:2, et/ou que l'on ajuste le rapport des phases entre d'une part les produits bruts nitrés à purifier et d'autre part le milieu de lavage dans la plage de 25:1 à 1:5, en particulier dans la plage de 10:1 à 1:2, de préférence dans la plage de 5:1 à 1:1.

14. Utilisation selon l'une des revendications précédentes, **caractérisée en ce que** le milieu de lavage est liquide dans les conditions du procédé, en particulier à des températures à partir de 5°C, en particulier à des températures à partir de 25°C, et sous la pression atmosphérique, et de préférence est à base aqueuse, est de préférence l'eau, et/ou que les produits bruts nitrés à purifier sont liquides dans les conditions du procédé, en particulier à des températures à partir de 5°C, en particulier à des températures à partir de 25°C et sous la pression atmosphérique, et/ou que les produits bruts nitrés à purifier proviennent de la nitration de composés aromatiques mono- ou polycycliques, en particulier le benzène, le toluène, le xylène ou les aromatiques halogénés, en particulier les benzènes chlorés, et/ou que les produits bruts nitrés à purifier sont des composés mono-, di- et trinitro-aromatiques éventuellement halogénés.
